# EUROPEAN PATENT APPLICATION

(11) **EP 4 169 527 A1**
(43) Date of publication of application: **26.04.2023**
(21) Application number: 21829834.7
(22) Date of filing: 21.06.2021
(51) Int. Cl.: A61K 39/00, A61K 39/02, A61K 39/39, A61P 31/04, A61P 37/04, A61P 43/00, C07H 5/06, A61K 9/127, A61K 47/24, A61K 47/26

(54) **ADJUVANT WITH TLR4 AGONIST ACTIVITY**

(30) Priority: 22.06.2020 JP 2020107194
(71) Applicant: Sumitomo Pharma Co., Ltd., Osaka 541-0045 (JP)
(72) Inventor: BAN, Hitoshi, Osaka-shi, Osaka 554-0022 (JP); IMAZAKI, Yusuke, Osaka-shi, Osaka 554-0022 (JP); TAKANASHI, Yosuke, Osaka-shi, Osaka 554-0022 (JP); FUKUSHIMA, Akihisa, Osaka-shi, Osaka 554-0022 (JP)
(74) Representative: Vossius & Partner Patentanwälte Rechtsanwälte mbB
(86) International application number: PCT/JP2021/023402
(87) International publication number: WO 2021/261444

(57) **Abstract**

The present invention relates to a compound useful as vaccine adjuvant, a manufacturing process thereof, a pharmaceutical composition comprising the compound, and use of the compound as vaccine adjuvant.

## Description

### TECHNICAL FIELD

The present invention relates to a compound useful as vaccine adjuvant, a manufacturing process thereof, a pharmaceutical composition comprising the compound, and use of the compound as vaccine adjuvant.

### BACKGROUND ART

A vaccine comprising a partial protein/peptide derived from some protein that microorganisms produce is advantageous in terms of the safety and the preparing process since it can be prepared through chemical synthesis or genetic engineering technique. On the other hand, such vaccine tends to have weaker efficacy than a live or inactivated vaccine prepared from raw bacteria, and sometimes an adjuvant is added to the vaccine to enhance immunogenicity. As an adjuvant for vaccine, alum has been often used, and recently a vaccine comprising 3-desacyl-4'-monophosphoryl lipid A (MPL) which is an agonist for Toll like receptor 4 (TLR4) as an adjuvant has been commercially approved (Non-Patent Literature 1).

It is known that TLR4 can prepare a heterodimer with MD-2 (myeloid differentiation factor 2), and activate the TLR4 pathway. Lipopolysaccharide (LPS) which is known as one of TLR4 agonists has a lipid A structure which is composed of phosphorylated disaccharide and fatty acid side chain (Non-Patent Literature 2), and it has been reported that the lipid A interacts with both of TLR4 and MD-2 to play an important role to form the heterodimer (Non-Patent Literature 3).

LPS can be transformed to the above-mentioned MPL by detoxification (Non-Patent Literature 1), and thus MPL is a mixture of plural compounds composed of phosphorylated disaccharide and fatty acid side chain, like LPS (Non-Patent Literature 4). The X-ray crystal structure analysis shows that the phosphate group interacts with both of TLR4 and MD-2 (Non-Patent Literature 3), and it is also known that the activity of MPL greatly decreases when the phosphate group is removed from the lipid A (Non-Patent Literature 2). And, it has been reported that the structure of fatty acid therein is also important on the TLR4 agonistic effect of MPL, and sometimes the effect of MPL may change to antagonistic effect depending on the structure of fatty acid side chain (Non-Patent Literature 2) .

Compounds derived from biological components such as MPL have manufacturing challenges, and thus some synthetic TLR4 agonists mimicking MPL have been studied. For example, Patent Literatures 1 and 2 reported that phosphorylated disaccharide structure which is a basic structure in aminoalkyl glucosaminide phosphate (AGP) was transformed to its phosphorylated monosaccharide structure, and then the phosphate group was an essential structure in AGP. And, Non-Patent Literature 5 also reported detailed studies about fatty acid side chain in AGP, in which the TLR4 agonistic effect of AGP was deactivated by transforming the fatty acid side chain, like the case of lipid A.

It is thought that synthetic TLR4 agonist which has a structure equipped with phosphate group such as AGP is disadvantageous in terms of manufacturing cost or storage stability, but there has been no report of vaccine adjuvants that are TLR4 agonist having saccharide and fatty acid side chain as main structures, but having no phosphate group, which still retain TLR4 agonistic effect, in the past research reports.

### PRIOR ART

### [Patent Reference]

[Patent Literature] WO 1998/050399
[Patent Literature 2] WO 2001/034617

### [Non-patent Reference]

[Non-Patent Literature 1] Cervarix interview form
[Non-Patent Literature 2] Microbes Infect. 2002 Jul; 4(8): 837-51.
[Non-Patent Literature 3] Nature. 2009 Apr 30; 458(7242): 1191-5.
[Non-Patent Literature 4] Regul Toxicol Pharmacol. 2002 Jun; 35(3): 398-413.
[Non-Patent Literature 5] J Biol Chem. 2004 Feb 6; 279(6): 4440-9

### Summary of Invention

### (Technical Problem)

The purpose of the present invention may be to provide a TLR4 agonist having a high adjuvant effect even having no phosphate group.

### (Solution to Problem)

The present inventors have extensively studied to find a TLR4 agonist having a high adjuvant effect, and then have found some compounds which retain TLR4 agonistic effect by suitably transforming their fatty acid side chain even having no phosphate group on the sugar structure. Further, the present inventors have found that these compounds have excellent adjuvant effect. Based upon the findings, the present invention has been achieved. According to the present invention, a TLR4 agonist derivative of the following formula (1) (hereinafter, also referred to as "the present compound") is provided.

The present invention is as described below.

### (Item 1)

A compound of formula (1): 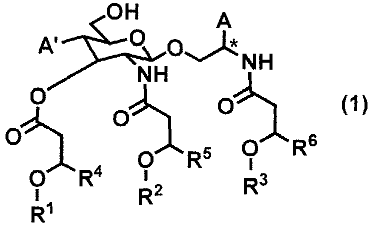 or a pharmaceutically acceptable salt thereof, wherein
A and A' are independently hydrogen, hydroxy, or - (CH₂)ₘ-COOH, provided that at least one of A or A' is - (CH₂)ₘ-COOH,
R¹ is -C(O)(CH₂)ₙ-X or -CH₂-(CH₂)ₙ-X,
R² is -C(O)(CH₂)ₒ-Y or -CH₂-(CH₂)ₒ-Y,
R³ is -C(O)(CH₂)ₚ-Z or -CH₂-(CH₂)ₚ-Z,
X, Y, and Z are independently methyl, C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy), or 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy), provided that at least one of X, Y, or Z is C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy) or 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy),
provided that when A is -COOH and the stereochemistry of * is S-configuration, Y is methyl,
R⁴, R⁵, and R⁶ are independently C₁₀₋₂₀ alkyl,
m is independently an integer of 0 - 6, and
n, o, and p are independently an integer of 5 - 20.

### (Item 2)

The compound of Item 1, or a pharmaceutically acceptable salt thereof, wherein m is 0.

### (Item 3)

The compound of Item 1 or 2, or a pharmaceutically acceptable salt thereof, wherein R¹ is -C(O)(CH₂)ₙ-X, R² is -C(O)(CH₂)ₒ-Y, and R³ is -C(O)(CH₂)ₚ-Z.

### (Item 4)

The compound of any one of Items 1 to 3, or a pharmaceutically acceptable salt thereof, wherein A is COOH.

### (Item 5)

The compound of any one of Items 1 to 4, or a pharmaceutically acceptable salt thereof, wherein A is COOH, and A' is hydroxy.

### (Item 6)

The compound of any one of Items 1 to 5, or a pharmaceutically acceptable salt thereof, wherein R⁴, R⁵, and R⁶ are independently C₁₀₋₁₂ alkyl.

### (Item 7)

The compound of Item 1 which is represented by formula (2) : 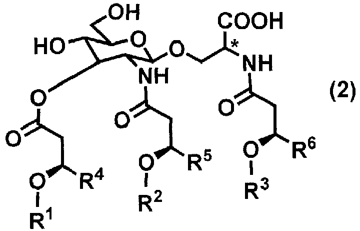 wherein
R¹ is -C(O)(CH₂)ₙ-X,
R² is -C(O)(CH₂)ₒ-Y,
R³ is -C(O)(CH₂)ₚ-Z,
X, Y, and Z are independently methyl, C₆₋₁₀ aryl, or 5-to 10-membered heteroaryl, provided that at least one of X, Y, or Z is C₆₋₁₀ aryl or 5- to 10-membered heteroaryl,
provided that when the stereochemistry of * is S-configuration, Y is methyl,
R⁴, R⁵, and R⁶ are independently C₁₀₋₁₂ alkyl, and
n, o, and p are independently an integer of 6 - 10, or a pharmaceutically acceptable salt thereof.

### (Item 8)

The compound of Item 1 which is represented by formula (3) : 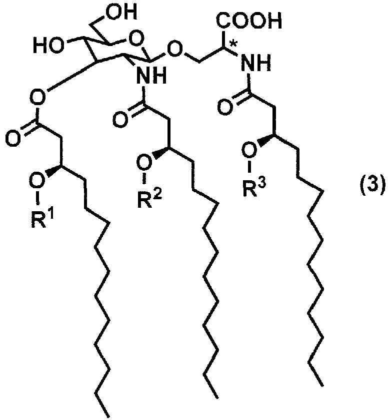 wherein
R¹ is -C(O)(CH₂)ₙ-X,
R² is -C(O)(CH₂)ₒ-Y,
R³ is -C(O)(CH₂)ₚ-Z,
X, Y, and Z are independently methyl, C₆₋₁₀ aryl, or 5-to 10-membered heteroaryl, provided that at least one of X, Y, or Z is C₆₋₁₀ aryl or 5- to 10-membered heteroaryl,
provided that when the stereochemistry of * is S-configuration, Y is methyl, and
n, o, and p are independently an integer of 6 - 10, or a pharmaceutically acceptable salt thereof.

### (Item 9)

The compound of Item 1 which is represented by formula (4) or formula (5): 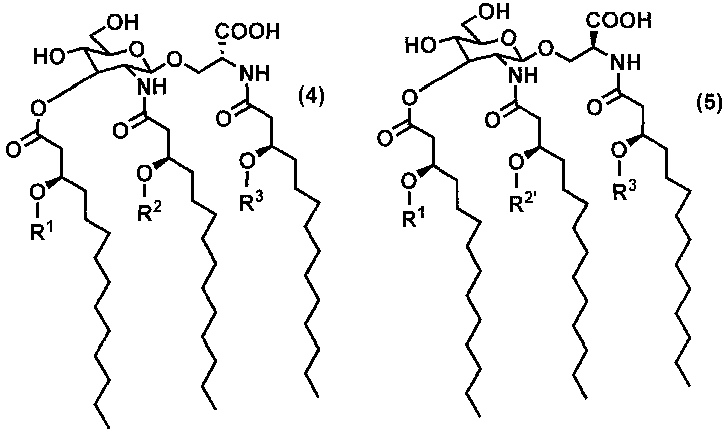 wherein
R¹ is -C(O)(CH₂)ₙ-X,
R² is -C(O)(CH₂)ₒ-Y,
R³ is -C(O)(CH₂)ₚ-Z,
R² is -C(O)(CH₂)ₒ-CH₃,
X, Y, and Z are independently methyl, C₆₋₁₀ aryl, or 5-to 10-membered heteroaryl, provided that at least one of X, Y, or Z in formula (4) is C₆₋₁₀ aryl or 5- to 10-membered heteroaryl, and at least one of X or Z in formula (5) is C₆₋₁₀ aryl or 5- to 10-membered heteroaryl, and
n, o, and p are independently an integer of 7 - 9, or a pharmaceutically acceptable salt thereof.

### (Item 10)

The compound of any one of Items 1 to 9, or a pharmaceutically acceptable salt thereof, wherein
X, Y, and Z are independently methyl or phenyl, provided that at least one of X, Y, or Z is phenyl,
provided that when A is -COOH and the stereochemistry of * is S-configuration, Y is methyl.

### (Item 11)

The compound of Item 1 which is selected from the following compound group:
(2*R*)-2-{[(3*R*)-3-(decanoyloxy)tetradecanoyl]amino}-3-{[3-{[(3*R*)-3-(decanoyloxy)tetradecanoyl]amino]-5-hydroxy-6-(hydroxymethyl)-4-({(3*R*)-3-[(9-phenylnonanoyl)oxy]tetradecanoyl}oxy)oxan-2-yl]oxy}propanoic acid (Example 1),
(2*S*)-2-{[(3*R*)-3-(decanoyloxy)tetradecanoyl]amino}-3-{[3-{[(3*R*)-3-(decanoyloxy)tetradecanoyl]amino}-5-hydroxy-6-(hydroxymethyl)-4-({(3*R*)-3-[(9-phenylnonanoyl)oxy]tetradecanoyl}oxy)oxan-2-yl]oxy}propanoic acid (Example 2),
(2*R*)-2-{[(3*R*)-3-(decanoyloxy)tetradecanoyl]amino}-3-{[4-{[(3*R*)-3-(decanoyloxy)tetradecanoyl]oxy}-5-hydroxy-6-(hydroxymethyl)-3-({(3*R*)-3-[(9-phenylnonanoyl)oxy]tetradecanoyl}amino)oxan-2-yl]oxy}propanoic acid (Example 3),
(2*R*)-2-({[(3*R*)-3-(9-phenylnonanoyl)oxy]tetradecanoyl}amino)-3-{[3-{[(3*R*)-3-(decanoyloxy)tetradecanoyl]amino}-5-hydroxy-6-(hydroxymethyl)-4-({(3*R*)-3-(decanoyloxy)tetradecanoyl}oxy)oxan-2-yl]oxy}propanoic acid (Example 4), and
(2*S*)-2-({[(3*R*)-3-(9-phenylnonanoyl)oxy]tetradecanoyl}amino)-3-{[3-{[(3*R*)-3-(decanoyloxy)tetradecanoyl]amino}-5-hydroxy-6-(hydroxymethyl)-4-({(3*R*)-3-(decanoyloxy)tetradecanoyl}oxy)oxan-2-yl]oxy}propanoic acid (Example 5),
or a pharmaceutically acceptable salt thereof.

### (Item 12)

A pharmaceutical composition comprising the compound of any one of Items 1 to 11, or a pharmaceutically acceptable salt thereof.

### (Item 13)

The pharmaceutical composition of Item 12, which is a lipid formulation.

### (Item 14)

The pharmaceutical composition of Item 12 or 13, wherein the lipid formulation is a liposome formulation including phospholipid.

### (Item 15)

The pharmaceutical composition of Item 14, wherein the phospholipid is 1,2-dimyristoyl-sn-glycero-3-phosphocholine and egg yolk phosphatidylglycerol.

### (Item 16)

The pharmaceutical composition of Item 14 or 15, wherein the lipid formulation comprises at least one additive selected form the group consisting of an inorganic acid, an inorganic acid salt, an organic acid, an organic acid salt, sugars, a buffering agent, an antioxidant, and polymers.

### (Item 17)

The pharmaceutical composition of any one of Items 12 to 16, which further comprises an antigen.

### (Item 18)

The pharmaceutical composition of Item 17, wherein the antigen is a pathogen-derived antigen.

### (Item 19)

A vaccine adjuvant comprising the compound of any one of Items 1 to 11, or a pharmaceutically acceptable salt thereof.

### (Item 20)

The vaccine adjuvant of Item 19, which is an adjuvant for infection vaccine.

### (Item 21)

A medicament for treating or preventing infection, comprising the compound of any one of Items 1 to 11, or a pharmaceutically acceptable salt thereof, which is used in combination with a pathogen-derived antigen.

### (Item 22)

The compound of any one of Items 1 to 11, or a pharmaceutically acceptable salt thereof, which is used as a vaccine adjuvant.

### (Item 23)

A method for enhancing specific immune response in a mammal to an antigen, comprising administering the compound of any one of Items 1 to 11 or a pharmaceutically acceptable salt thereof to the mammal.

### (Item 24)

Use of the compound of any one of Items 1 to 11 or a pharmaceutically acceptable salt thereof in the preparation of a vaccine adjuvant.

### (Item 25)

A kit comprising
a) the compound of Item 1 or a pharmaceutically acceptable salt thereof, or a pharmaceutical composition comprising the compound of Item 1 or a pharmaceutically acceptable salt thereof; and
b) a pharmaceutical composition comprising an antigen.

### (Item 26)

The kit of Item 25, wherein the antigen is a pathogen-derived antigen.

### (Effect of the Invention)

The present compound has acquired TLR4 agonistic effect by suitably transforming the fatty acid side chain even having no phosphate group on the sugar structure, which has a high adjuvant effect. The present compound has a high storage stability since it has no phosphate group on the sugar structure, and the manufacturing cost can be lowered since there is no process of introducing a phosphate group. Thus, the present compound is very useful as a vaccine adjuvant.

### BRIEF DESCRIPTION OF DRAWINGS

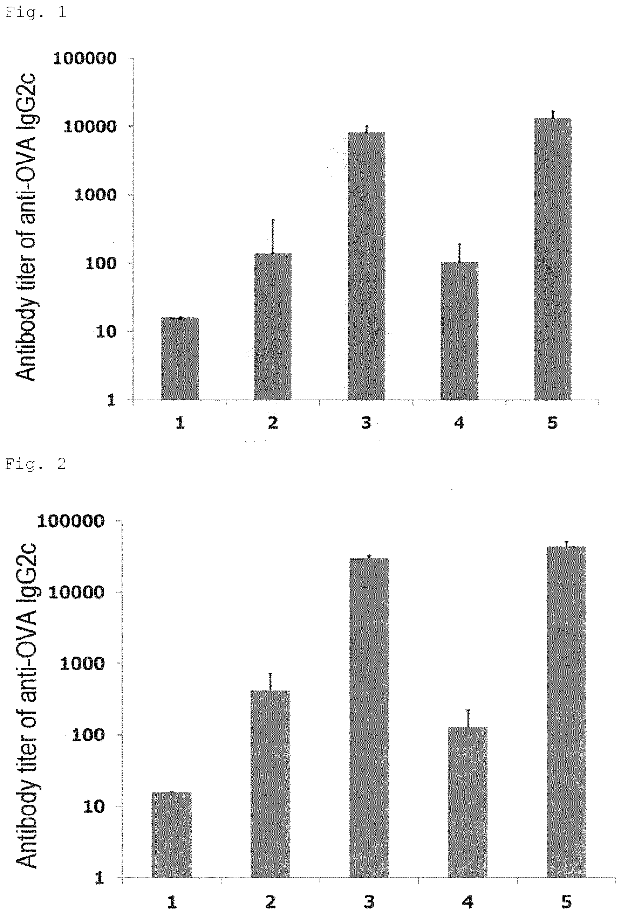

FIG. 1 is a graph showing the results of measuring OVA-specific IgG2c in immunized mouse serum in mice to which the formulation prepared in Example 6 or 7 was intramuscularly administered, by ELISA in Test 3. The vertical axis indicates the antibody titer of OVA-specific IgG2c in serum. The horizontal axis indicates the administered sample (administered dose in parentheses). 1: negative control group (phosphate buffered saline), 2: Example 6 administration group (the compound of Example 1, 10 µg/mouse), 3: Example 6 administration group (the compound of Example 1, 100 pg/mouse), 4: Example 7 administration group (the compound of Example 2, 10 pg/mouse), 5: Example 7 administration group (the compound of Example 2, 100 µg/mouse).
FIG. 2 is a graph showing the results of measuring OVA-specific IgG2c in immunized mouse serum in mice to which the formulation prepared in Reference Example 16 or Example 6 was intramuscularly administered, by ELISA in Test 3. The vertical axis indicates the antibody titer of OVA-specific IgG2c in serum. The horizontal axis indicates the administered sample (administered dose in parentheses). 1: negative control group (phosphate buffered saline), 2: Reference example 16 administration group (the compound of Reference example 9, 10 pg/mouse), 3: Reference example 16 administration group (the compound of Reference example 9, 100 pg/mouse), 4: Example 6 administration group (the compound of Example 1, 10 µg/mouse), 5: Example 6 administration group (the compound of Example 1, 100 µg/mouse).

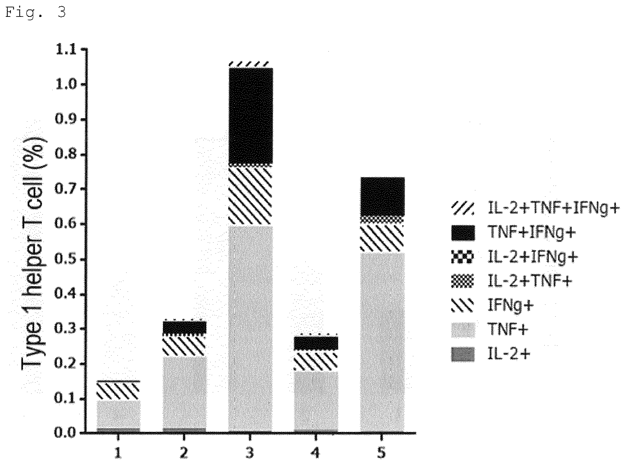

FIG. 3 is a graph showing the results of the evaluation performed in Test 4, showing the percentage of type 1 helper T cell in the spleen cell of the mouse to which the formulation prepared in Example 6 or 7 was intramuscularly administered. The horizontal axis is the same as FIG. 1.

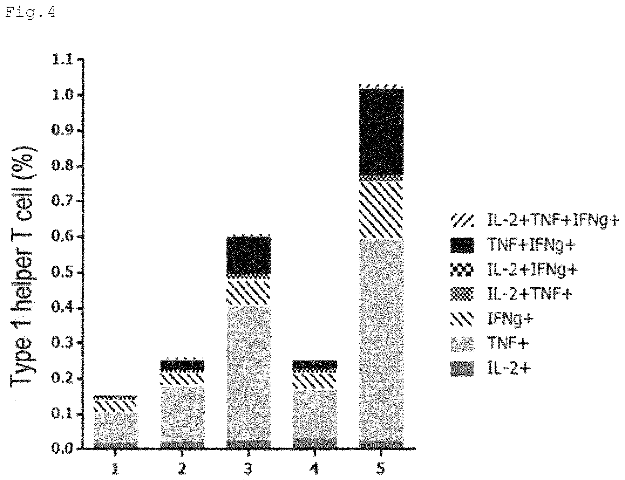

FIG. 4 is a graph showing the results of the evaluation performed in Test 4, showing the percentage of type 1 helper T cell in the spleen cell of the mouse to which the formulation prepared in Reference example 16 or Example 6 was intramuscularly administered. The horizontal axis is the same as FIG. 2.

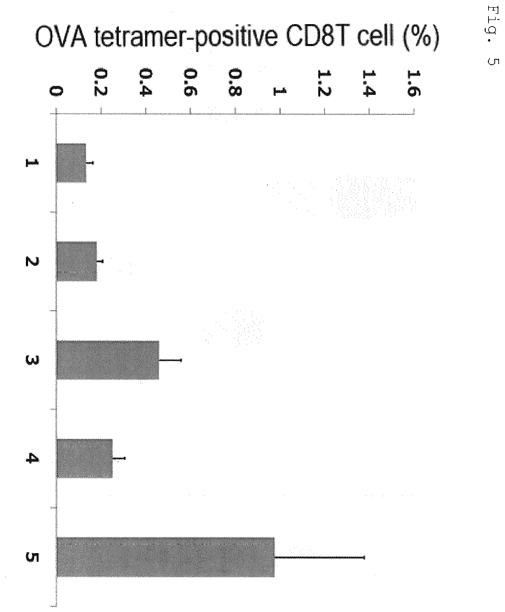

FIG. 5 is a graph showing the results of the evaluation performed in Test 4, showing the percentage of OVA tetramer-positive CD8T cell in the spleen cell of the mouse to which the formulation prepared in Example 6 or Example 7 was intramuscularly administered. The horizontal axis is the same as FIG. 1.

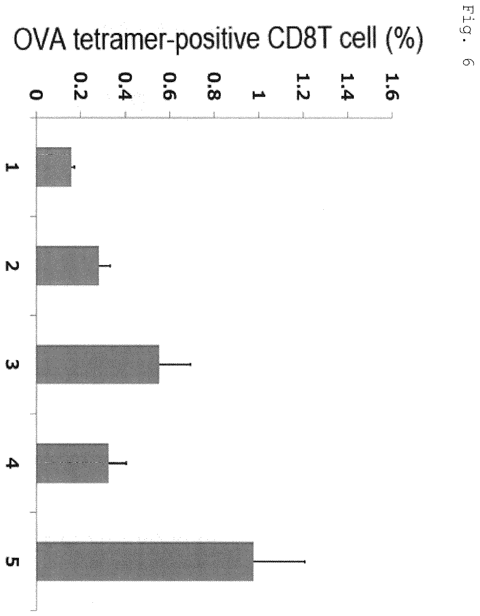

FIG. 6 is a graph showing the results of the evaluation performed in Test 4, showing the percentage of OVA tetramer-positive CD8T cell in the spleen cell of the mouse to which the formulation prepared in Reference Example 16 or Example 6 was intramuscularly administered. The horizontal axis is the same as FIG. 2.

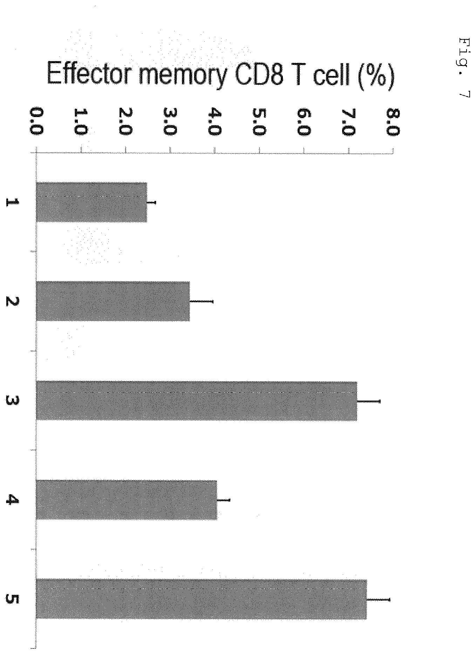

FIG. 7 is a graph showing the results of the evaluation performed in Test 4, showing the percentage of effector memory CD8 T cell in the spleen cell of the mouse to which the formulation prepared in Example 6 or 7 was intramuscularly administered. The horizontal axis is the same as FIG. 1.

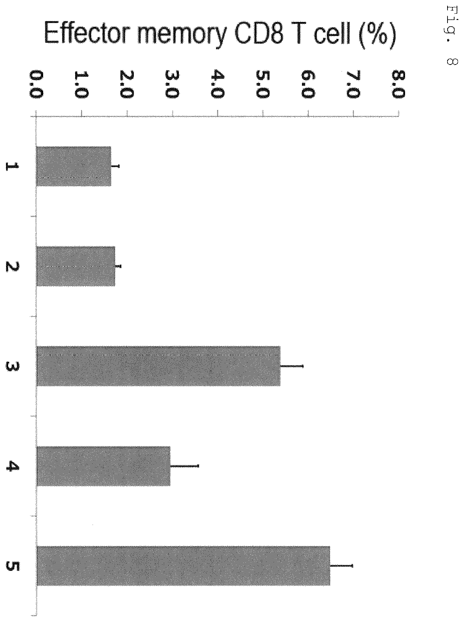

FIG. 8 is a graph showing the results of the evaluation performed in Test 4, showing the percentage of effector memory CD8 T cell in the spleen cell of the mouse to which the formulation prepared in Reference Example 16 or Example 6 was intramuscularly administered. The horizontal axis is the same as FIG. 2.

### DESCRIPTION OF EMBODIMENTS

Hereinafter, terms used herein are explained as follows.

The number of substituents that are defined posterior to "optionally-substituted" or "substituted" should not be limited, if it is possible to be substituted. Unless otherwise specified, the definition of each substituent group also extends over the case of partially-including the substituent group or the case of the substituent group existing on another substituent group.

The "halogen" used herein includes, for example, fluorine, chlorine, bromine, and iodine. It is preferably fluorine or chlorine, more preferably fluorine.

The "C₁₋₆ alkyl" means straight or branched chain saturated hydrocarbon group having 1 to 6 carbon atoms. The C₁₋₆ alkyl includes preferably "C₁₋₄ alkyl", more preferably "C₁₋₃ alkyl". The "C₁₋₆ alkyl" includes, for example, methyl, ethyl, propyl, 1-methylethyl, butyl, 2-methylpropyl, 1-methylpropyl, 1,1-dimethylethyl, pentyl, 3-methylbutyl, 2-methylbutyl, 2,2-dimethylpropyl, 1-ethylpropyl, 1,1-dimethylpropyl, hexyl, 4-methylpentyl, 3-methylpentyl, 2-methylpentyl, and 1-methylpentyl, and the "C₁₋₄ alky" includes the examples of the "C₁₋₆ alkyl" provided that the number of carbon atoms is 1 - 4. The "C₁₋₃ alkyl" includes the examples of the "C₁₋₆ alkyl" provided that the number of carbon atoms is 1 - 3.

The "C₁₋₆ alkoxy" means "C₁₋₆ alkyloxy", and the part "C₁₋₆ alkyl" is as defined in the said "C₁₋₆ alkyl". The "C₁₋₆ alkoxy" includes preferably "C₁₋₄ alkoxy", more preferably "C₁₋₃ alkoxy". The "C₁₋₆ alkoxy" includes, for example, methoxy, ethoxy, propoxy, 1-methylethoxy, butoxy, 2-methylpropoxy, 1-methylpropoxy, 1,1-dimethylethoxy, pentyloxy, 3-methylbutoxy, 2-methylbutoxy, 2,2-dimethylpropoxy, 1-ethylpropoxy, 1,1-dimethylpropoxy, hexyloxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, and 1,2-dimethylbutoxy, and the "C₁₋₄ alkoxy" includes the examples of the "C₁₋₆ alkoxy" provided that the number of carbon atoms is 1 - 4. The "C₁₋₃ alkoxy" includes the examples of the "C₁₋₆ alkoxy" provided that the number of carbon atoms is 1 - 3.

The "C₁₀₋₂₀ alkyl" means straight or branched chain saturated hydrocarbon group having 10 - 20 carbon atoms. The C₁₀₋₂₀ alkyl includes preferably "C₁₀₋₁₅ alkyl", more preferably "C₁₀₋₁₂ alkyl", and even more preferably straight chain "C₁₀₋₁₂ alkyl". The straight chain "C₁₀₋₂₀ alkyl" includes, for example, decyl, undecyl, dodecyl, tridecyl, tetradecyl, pentadecyl, hexadecyl, heptadecyl, octadecyl, nonadecyl, and eicosyl, and the "C₁₀₋₁₅ alkyl" includes the above examples of "C₁₀₋₂₀ alkyl" wherein the number of carbon atoms are 10 - 15. The "C₁₀₋₁₂ alkyl" includes the above examples of "C₁₀₋₂₀ alkyl" wherein the number of carbon atoms are 10 - 12.

The "C₆₋₁₀ aryl" means aromatic hydrocarbon having 6 - 10 carbon atoms. The "C₆₋₁₀ aryl" includes, for example, phenyl, 1-naphthyl, and 2-naphthyl, and more preferably phenyl.

The "5- to 10-membered heteroaryl" means a monocyclic 5- to 7-membered aromatic or bicyclic 8- to 10-membered aromatic heterocyclyl group comprising 1 to 4 atoms selected independently from nitrogen atom, oxygen atom, and sulfur atom. The "5- to 10-membered heteroaryl" includes preferably "5- to 7-membered heteroaryl", more preferably 5- to 7-membered aromatic heterocyclyl having one or more nitrogen atom in the ring ("5- to 7-membered nitrogen-containing heteroaryl").

The "5- to 10-membered heteroaryl" includes, for example, pyridyl, pyridazinyl, isothiazolyl, pyrrolyl, furyl, thienyl, thiazolyl, imidazolyl, pyrimidinyl, thiadiazolyl, pyrazolyl, oxazolyl, isoxazolyl, pyrazinyl, triazinyl, triazolyl, imidazolidinyl, oxadiazolyl, triazolyl, tetrazolyl, indolyl, indazolyl, quinolyl, isoquinolyl, benzofuranyl, benzothienyl, benzoxazolyl, benzothiazolyl, benzisoxazolyl, benzisothiazolyl, benzotriazolyl, benzimidazolyl, and 6,11-dihydrodibenzo [b,e]thiepinyl, preferably pyridyl, pyrimidinyl, quinolyl, and isoquinolyl, and more preferably pyridyl.

The "5- to 7-membered heteroaryl" includes, for example, monocyclic groups in the above examples of "5- to 10-membered heteroaryl". The "5- to 7-membered nitrogen-containing heteroaryl" includes, for example, nitrogen-containing monocyclic groups in the above examples of "5-to 10-membered heteroaryl".

The C₆₋₁₀ aryl used herein may have 1 - 5 substituents selected independently from hydroxy, C₁₋₆ alkyl, halogen, cyano, and C₁₋₆ alkoxy. The 5- to 10-membered heteroaryl may have 1 - 4 substituents selected independently from hydroxy, C₁₋₆ alkyl, halogen, cyano, and C₁₋₆ alkoxy. The number of the substituents which may be on the C₆₋₁₀ aryl or the 5- to 10-membered heteroaryl is preferably 1 - 3, more preferably 1 - 2, and even more preferably 1.

In the present compounds of formulae (1) - (5), preferred A, A', R¹, R², R^{2'}, R³, X, Y, Z, R⁴, R⁵, R⁶, m, n, o, and p are shown below, but the technical scope of the present invention is not limited to the scope of compounds listed below.

A includes preferably hydrogen, hydroxy, and - (CH₂)ₘ-COOH, more preferably -(CH₂)ₘ-COOH, and even more preferably -COOH.

A' includes preferably hydrogen, hydroxy, and -(CH₂)ₘ-COOH, more preferably hydroxy.

The combination of A and A' includes preferably a combination of A and A' wherein at least one of A and A' is -(CH₂)ₘ-COOH. More preferably, the combination includes a combination wherein A is -(CH₂)ₘ-COOH, and A' is hydroxy. Even more preferably, the combination includes a combination wherein A is -COOH, and A' is hydroxy.

R¹ includes preferably -C(O) (CH₂)ₙ-X and -CH₂-(CH₂)ₙ-X, more preferably -C(O)(CH₂)ₙ-X.

R² includes preferably -C(O)(CH₂)ₒ-Y and -CH₂-(CH₂)ₒ-Y, more preferably -C(O)(CH₂)ₒ-Y.

R^{2'} includes preferably -C(O)(CH₂)ₒ-CH₃.

R³ includes preferably -C(O)(CH₂)ₚ-Z and -CH₂-(CH₂)ₚ-Z, more preferably -C(O)(CH₂)ₚ-Z.

X includes preferably methyl, C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy), and 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy), more preferably methyl and C₆₋₁₀ aryl, more preferably methyl and phenyl, and even more preferably phenyl.

Y includes preferably methyl, C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy), and 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy), more preferably methyl and C₆₋₁₀ aryl, even more preferably methyl and phenyl, and even much more preferably methyl.

Z includes preferably methyl, C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy), and 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy), more preferably methyl and C₆₋₁₀ aryl, even more preferably methyl and phenyl, and even much more preferably methyl.

The combination of X, Y, and Z includes preferably a combination of X, Y, and Z wherein at least one of X, Y, and Z is C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy) or 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy), provided that when A is -COOH and the stereochemistry of * is S-configuration, the combination includes a combination of X, Y, and Z wherein Y is methyl, and at least one of X and Z is C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy) or 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy) . More preferably, the combination of X, Y, and Z includes a combination of X, Y, and Z wherein at least one of X, Y, and Z is C₆₋₁₀ aryl or 5- to 10-membered heteroaryl, provided that when A is -COOH and the stereochemistry of * is S-configuration, the combination includes a combination of X, Y, and Z wherein Y is methyl, and at least one of X and Z is C₆₋₁₀ aryl or 5- to 10-membered heteroaryl.

Even more preferably, the combination of X, Y, and Z includes a combination of X, Y, and Z wherein at least one of X, Y, and Z is phenyl, provided that when A is -COOH and the stereochemistry of * is S-configuration, the combination includes a combination of X, Y, and Z wherein Y is methyl, and at least one of X and Z is phenyl.

Even much more preferably, the combination of X, Y, and Z includes a combination of X, Y, and Z wherein X is phenyl, and Y and Z are methyl.

R⁴, R⁵, and R⁶ include independently, preferably C₁₀₋₂₀ alkyl, more preferably C₁₀₋₁₅ alkyl, more preferably C₁₀₋₁₂ alkyl, and even more preferably C₁₁ alkyl.
m includes preferably an integer of 0 - 6, more preferably an integer of 0 - 1, and even more preferably 0.
n, o, and p include independently, preferably an integer of 5 - 20, more preferably an integer of 6 - 10, even more preferably 7 - 9, and even much more preferably 8.

Preferred embodiments of the compounds of formula (1) include the following compounds or a pharmaceutically acceptable salt thereof.

In an embodiment, the present compound of formula (1) includes the following (A).
(A)

A compound of formula (1) or a pharmaceutically acceptable salt thereof, wherein
A is hydrogen, hydroxy, or -(CH₂)ₘ-COOH;
A' is hydrogen, hydroxy, or -(CH₂)ₘ-COOH;
provided that at least one of A or A' is -(CH₂)ₘ-COOH;
R¹ is -C(O)(CH₂)ₙ-X or -CH₂-(CH₂)ₙ-X;
R² is -C(O)(CH₂)ₒ-Y or -CH₂-(CH₂)ₒ-Y;
R³ is -C(O)(CH₂)ₚ-Z or -CH₂-(CH₂)ₚ-Z;
X, Y, and Z are independently methyl, C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy), or 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy);
provided that at least one of X, Y, or Z is C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy) or 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy),
provided that when A is -COOH and the stereochemistry of * is S-configuration, Y is methyl;
R⁴, R⁵, and R⁶ are independently C₁₀₋₂₀ alkyl;
m is independently an integer of 0 - 6; and
n, o, and p are independently an integer of 5 - 20.

In an embodiment, the present compound of formula (1) includes the following (B).
(B)

A compound of formula (1) or a pharmaceutically acceptable salt thereof, wherein
A is -COOH;
A' is hydroxy;
R¹ is -C(O)(CH₂)ₙ-X;
R² is -C(O)(CH₂)ₒ-Y;
R³ is -C(O)(CH₂)ₚ-Z;
X, Y, and Z are independently methyl, C₆₋₁₀ aryl, or 5-to 10-membered heteroaryl;
provided that at least one of X, Y, or Z is C₆₋₁₀ aryl or 5- to 10-membered heteroaryl,
provided that when the stereochemistry of * is S-configuration, Y is methyl;
R⁴, R⁵, and R⁶ are independently C₁₀₋₁₂ alkyl; and
n, o, and p are independently an integer of 6 - 10.

In an embodiment, the present compound of formula (1) includes the following (C).
(C)

A compound of formula (1) or a pharmaceutically acceptable salt thereof, wherein
A is -COOH;
A' is hydroxy;
R¹ is -C(O) (CH₂)ₙ-X;
R² is -C(O) (CH₂)ₒ-Y;
R³ is -C(O) (CH₂)ₚ-Z;
X, Y, and Z are independently methyl, C₆₋₁₀ aryl, or 5-to 10-membered heteroaryl;
provided that at least one of X, Y, or Z is C₆₋₁₀ aryl or 5- to 10-membered heteroaryl,
provided that when the stereochemistry of * is S-configuration, Y is methyl,
R⁴, R⁵, and R⁶ are independently undecyl; and
n, o, and p are independently an integer of 6 - 10.

In an embodiment, the present compound of formula (1) includes the following (D).
(D)

A compound of formula (1) or a pharmaceutically acceptable salt thereof, wherein
A is -COOH;
A' is hydroxy;
R¹ is -C(O)(CH₂)ₙ-X;
R² is -C(O)(CH₂)ₒ-Y;
R³ is -C(O)(CH₂)ₚ-Z;
X, Y, and Z are independently methyl, C₆₋₁₀ aryl, or 5-to 10-membered heteroaryl;
provided that at least one of X, Y, or Z is C₆₋₁₀ aryl and 5- to 10-membered heteroaryl,
provided that when the stereochemistry of * is S-configuration, Y is methyl;
R⁴, R⁵, and R⁶ are independently undecyl; and
n, o, and p are independently an integer of 7 - 9.

In an embodiment, the compound of formula (1) includes the following compound group:
(2*R*)-2-{[(3*R*)-3-(decanoyloxy)tetradecanoyl]amino}-3-{[3-{[(*3*R)-3-(decanoyloxy)tetradecanoyl]amino}-5-hydroxy-6-(hydroxymethyl)-4-({(3*R*)-3-[(9-phenylnonanoyl)oxy]tetradecanoyl}oxy)oxan-2-yl]oxy}propanoic acid (Example 1);
(2*S*)-2-{[(3*R*)-3-(decanoyloxy)tetradecanoyl]amino}-3-{[3-{[(3*R*)**-**3-(decanoyloxy)tetradecanoyl]amino}-5-hydroxy-6-(hydroxymethyl)-4-({(3*R*)-3-[(9-phenylnonanoyl)oxy]tetradecanoyl}oxy)oxan-2-yl]oxy}propanoic acid (Example 2);
(2*R*)-2-{[(3*R*)-3-(decanoyloxy)tetradecanoyl]amino}-3-{[4-{[(3*R*)-3-(decanoyloxy)tetradecanoyl]oxy}-5-hydroxy-6-(hydroxymethyl)-3-({(3*R*)-3-[(9-phenylnonanoyl)oxy]tetradecanoyl}amino)oxan-2-yl]oxy}propanoic acid (Example 3);
(2*R*)-2-({[(3*R*)-3-(9-phenylnonanoyl)oxy]tetradecanoyl}amino)-3-{[3-{[(3*R*)-3-(decanoyloxy)tetradecanoyl]amino}-5-hydroxy-6-(hydroxymethyl)-4-({(3*R*)-3-(decanoyloxy)tetradecanoyl}oxy)oxan-2-yl]oxy}propanoic acid (Example 4); and
(2*S*)-2-({[(3*R*)-3-(9-phenylnonanoyl)oxy]tetradecanoyl}amino)-3-{[3-{[(3*R*)-3-(decanoyloxy)tetradecanoyl]amino}-5-hydroxy-6-(hydroxymethyl)-4-({(3*R*)-3-(decanoyloxy)tetradecanoyl}oxy)oxan-2-yl]oxy}propanoic acid (Example 5).

Hereinafter, the processes to prepare the compound of the present invention are explained, but the processes of the present invention should not be limited thereto.

The present compound of formula (1) can be prepared, for example, according to Processes 1 to 3 described below.

### Process 1

The compound of formula (1) can be prepared, for example, by the following process. 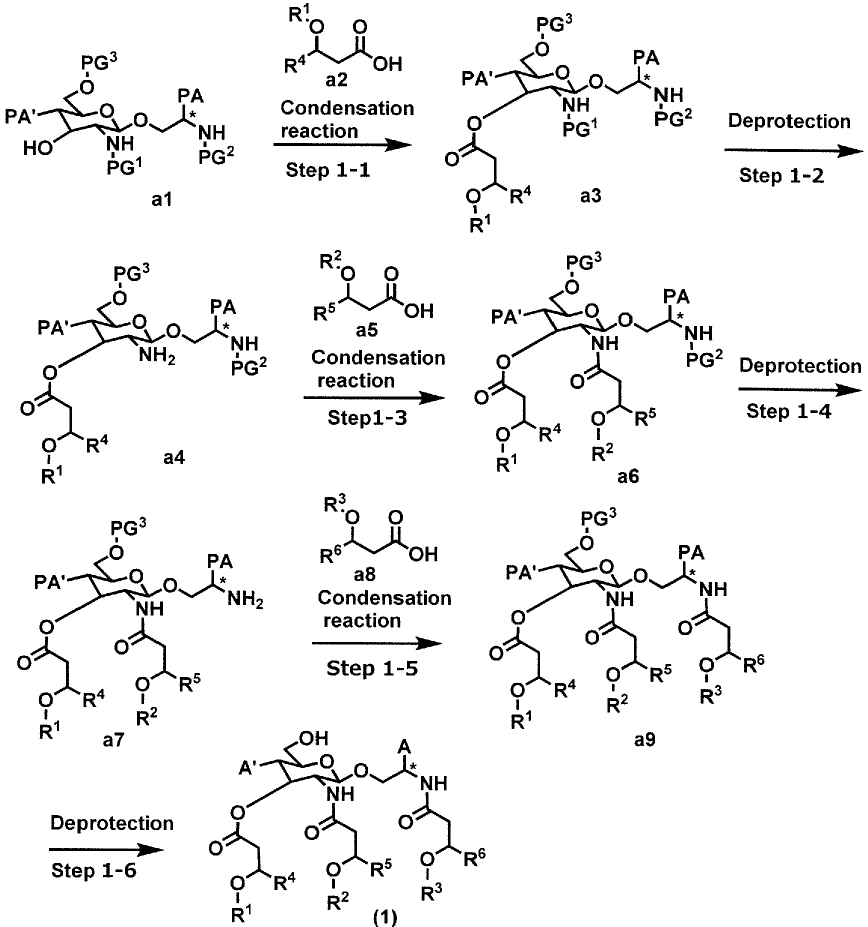 wherein R¹, R², R³, R⁴, R⁵, R⁶, A, A', and m are as defined in Item 1; PA and PA' are independently hydrogen, hydroxy, O-PG⁴, or (CH₂)ₘ-C(O)O-PG⁵; PG¹ and PG² are independently an amino-protecting group; PG³ and PG⁴ are independently a hydroxy-protecting group; and PG⁵ is a carboxyl-protecting group.

The protecting groups represented by the above PG¹, PG², PG³, PG⁴, and PG⁵ may include protecting groups described in Protective Groups in Organic Synthesis (edited by Theodora W. Greene, Peter G. M. Wuts, issued by John Wiley & Sons, Inc., 1999). Compound a1 can be prepared, for example, by the process described in WO 98/50399. Compounds a2, a5, and a8 can be prepared by Processes 2 and 3 which are described later.

### (Step 1-1)

Compound a3 can be prepared by reacting Compound a1 with Compound a2 in a suitable solvent in the presence of a suitable condensation agent. The condensation agent used herein may be selected from the exemplified condensation agents listed later, which includes preferably carbodiimides, more preferably 1-[3-(dimethylamino)propyl]-3-ethylcarbodiimide methiodide. Preferably, the condensation reaction may be done along with N,N-dimethyl-4-aminopyridine (DMAP) or 4-pyrrolidinopyridine which is a promoter in condensation reaction. The solvent used herein may be selected from the exemplified solvents listed later, which includes preferably chloroform and dichloromethane. The reaction time is generally 5 minutes to 48 hours, preferably 1 to 24 hours. The reaction temperature is generally -78°C to 100°C, preferably 0°C to 50°C.

### (Step 1-2)

Compound a4 can be prepared by deprotecting the amino-protecting group PG¹ of Compound a3. The step can be done according to a known method described in Protective Groups in Organic Synthesis (edited by Theodora W. Greene, Peter G. M. Wuts, issued by John Wiley & Sons, Inc., 1999) and the like.

### (Step 1-3)

Compound a6 can be prepared by reacting Compound a4 with Compound as in a suitable solvent in the presence of a suitable condensation agent. The condensation agent used herein may be selected from the exemplified reagents listed later, which includes preferably 1-ethoxycarbonyl-2-ethoxy-1,2-dihydroquinoline. The solvent used herein may be selected from the exemplified solvents listed later, which includes preferably chloroform and dichloromethane. The reaction time is generally 5 minutes to 48 hours, preferably 1 to 24 hours. The reaction temperature is generally -78°C to 100°C, preferably 0°C to 50°C.

### (Step 1-4)

Compound a7 can be prepared by deprotecting the amino-protecting group PG² of Compound a6. The step can be done according to a known method described in Protective Groups in Organic Synthesis (edited by Theodora W. Greene, Peter G. M. Wuts, issued by John Wiley & Sons, Inc., 1999) and the like.

### (Step 1-5)

Compound a9 can be prepared by reacting Compound a7 with Compound a8 by the method according to the above Step 1-3.

### (Step 1-6)

The compound of formula (1) can be prepared by deprotecting the protecting group PG³ in Compound a9, and by deprotecting PG⁴ and/or PG⁵ when PA and PA' have O-PG⁴ and/or C(O)OPG⁵. The step can be done according to a known method described in Protective Groups in Organic Synthesis (edited by Theodora W. Greene, Peter G. M. Wuts, issued by John Wiley & Sons, Inc., 1999) and the like.

The above condensation reactions with Compounds a2, a5, and a8 may be done simultaneously or in a different order, depending on the substituents. For example, when R² and R³ are the same and R⁵ and R⁶ are the same, Compound a9 can be prepared from Compound a3 in two steps of Step 1-2 and Step 1-3 as shown below, by using the same protecting group in PG¹ and PG².

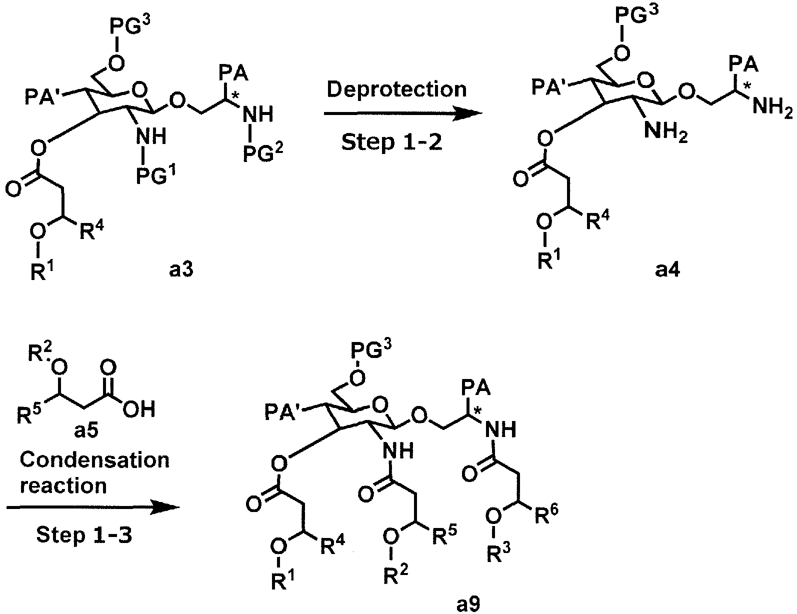

### Process 2

Compound a2 drawn in Process 1 wherein R¹ in formula (1) is -C(O) (CH₂)ₙ-X can be prepared, for example, by the following process. Compound a5 wherein R² is -C(O) (CH₂)ₒ-Y and Compound a8 wherein R³ is -C(O)(CH₂)ₚ-Z can be also prepared in the same process. 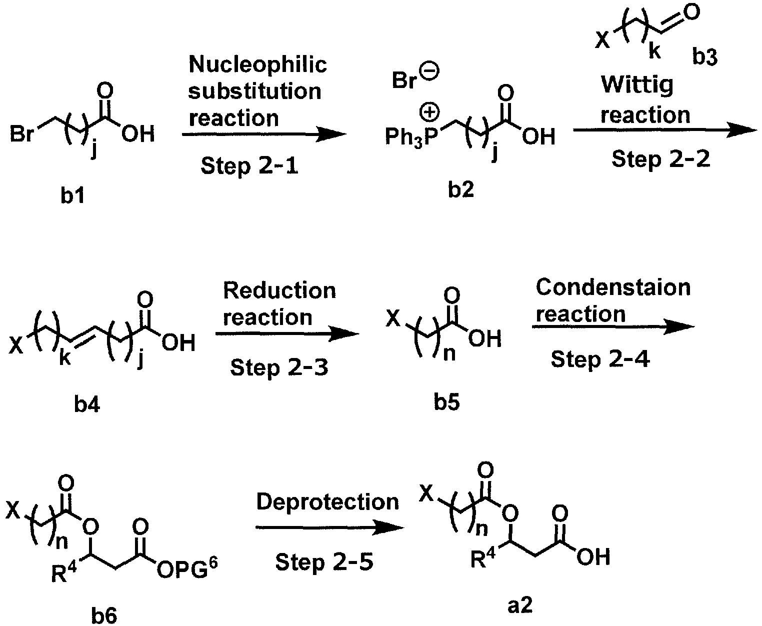 wherein X, R⁴, and n are as defined in Item 1; j is an integer of 0 - 18; k is n-j-2; and PG⁶ is a carboxyl-protecting group.

Step 2-1 - Step 2-3 are, for example, a known method according to the process described in US2008/0188566. And, Step 2-4 and Step 2-5 are, for example, a known method according to the process described in WO2004/062599.

### Process 3

Compound a2 drawn in Process 1 wherein R¹ in formula (1) is -CH₂-(CH₂)ₙ-X can be prepared, for example, by the following process. Compound a5 wherein R² is -CH₂(CH₂)ₒ-Y and Compound a8 wherein R³ is -CH₂(CH₂)ₚ-Z can be also prepared in the same process. 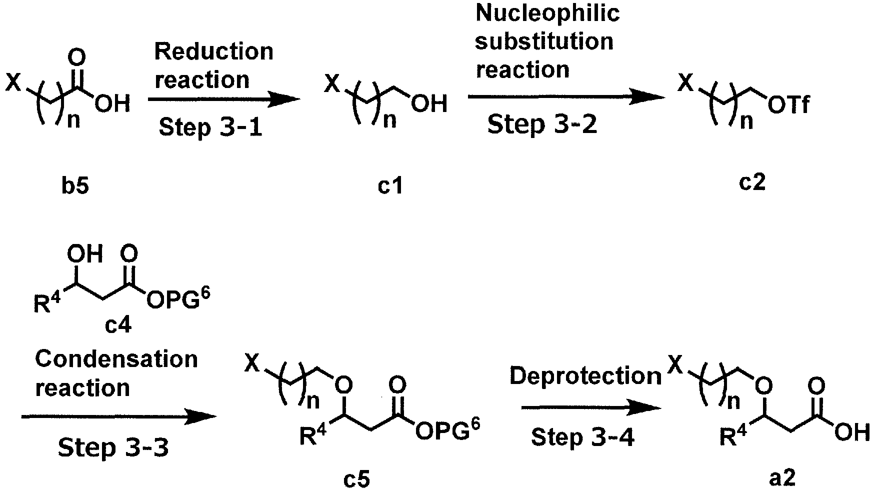 wherein X, R⁴, and n are as defined in Item 1; PG⁶ is a carboxyl-protecting group.

Step 3-1 is a reduction reaction from carboxylic acid to alcohol, which can be done, for example, according to the process disclosed in Series of Experimental Chemistry 5th edition, Vol. 14, p11- 16 (Jikken Kagaku Kouza, edited by the Chemical Society of Japan, 2005). And, Step 3-2 - Step 3-4 are, for example, a known method according to the process described in WO 01/36433.

Compound a2 can be also prepared by the method described in Bioorg Med Chem Lett. 2015 Feb 1; 25(3): 547-53.

The base used in each step of the above processes should be suitably selected based on the reaction, the starting compound, etc., which includes alkaline bicarbonates such as sodium bicarbonate, and potassium bicarbonate; alkaline carbonate such as sodium carbonate, and potassium carbonate; metallic hydrides such as sodium hydride, and potassium hydride; alkaline metal hydroxides such as sodium hydroxide, and potassium hydroxide; alkaline metal alkoxides such as sodium methoxide, and sodium t-butoxide; organic metal bases such as butyllithium, and lithium diisopropylamide; and organic bases such as triethylamine, diisopropylethylamine, pyridine, 4-dimethylaminopyridine (DMAP), and 1,8-diazabicyclo[5.4.0]undec-7-ene (DBU).

The condensation agent should be suitably selected depending on starting compounds, etc., which includes, for example, phosphates such as diethyl cyanophosphate and diphenylphosphoryl azide; carbodiimides such as 1-ethyl-3-(3-dimethylaminopropyl)-carbodiimide hydrochloride (WSC·HCl) and dicyclohexylcarbodiimide (DCC), and 1-[3-(dimethylamino)propyl]-3-ethylcarbodiimide methiodide; combinations of a disulfide such as 2,2'-dipyridyldisulfide and a phosphine such as triphenylphosphine; phosphorus halides such as N,N'-bis(2-oxo-3-oxazolidinyl)phosphinic chloride (BOPCl); combinations of an azodicarboxylate diester such as diethyl azodicarboxylate and a phosphine such as triphenylphosphine; 2-halo-1-lower alkylpyridinium halides such as 2-chloro-1-methylpyridinium iodide; 1,1'-carbonyldiimidazole (CDI); 1-ethoxycarbonyl-2-ethoxy-1,2-dihydroquinoline (EEDQ); diphenylphosphoryl azide (DPPA); diethylphosphoryl cyanide (DEPC); tetrafluoroborates such as 2-(1H-benzotriazol-1-yl)-1,1,3,3-tetramethyluronium tetrafluoroborate (TBTU) and 2-chloro-1,3-dimethylimidazolidinium tetrafluoroborate (CIB); phosphates such as 2-(1H-benzotriazol-1-yl)-1,1,3,3-tetramethyluronium hexafluorophosphate (HBTU), benzotriazol-1-yloxytris(dimethylamino)phosphonium hexafluorophosphate (BOP), benzotriazol-1-yloxytris(pyrrolidino)phosphonium hexafluorophosphate (PYBOP), and 2-(7-aza-1H-benzotriazol-1-yl)-1,1,3,3-tetramethyluronium hexafluorophosphate (HATU).

The solvent used in each step of the above processes should be suitably selected based on the reaction, the starting compound, etc., which includes, for example, alcohol solvents such as methanol, ethanol, and isopropanol; ketone solvents such as acetone and methylketone; halogenated hydrocarbon solvents such as methylene chloride and chloroform; ether solvents such as tetrahydrofuran (THF) and dioxane; aromatic hydrocarbon solvents such as toluene and benzene; aliphatic hydrocarbon solvents such as hexane and heptane; ester solvents such as ethyl acetate and propyl acetate; amide solvents such as N,N-dimethylformamide (DMF) and N-methyl-2-pyrrolidone; sulfoxide solvents such as dimethylsulfoxide (DMSO); nitrile solvents such as acetonitrile. The solvent used herein may be one of these solvents or a mixture of two or more solvents selected from these solvents. And, if possible in the reaction, an organic base or an organic acid may be used as a solvent used herein.

In addition, each intermediate or each final product in the above preparation processes can be also transformed to another compound of the present invention by suitably modifying its functional group, especially extending various side-chains from amino, hydroxy, carbonyl, halogen, etc.; and optionally making the above-mentioned protection and deprotection if necessary. The modification of functional group and the extension of side-chain can be done by a conventional method (for example, see Comprehensive Organic Transformations, R. C. Larock, John Wiley & Sons Inc. (1999), etc.).

The "pharmaceutically acceptable salt" includes an acid addition salt and a base addition salt. For example, the acid addition salt includes an inorganic acid salt such as hydrochloride, hydrobromide, sulfate, hydroiodide, nitrate, and phosphate; and an organic acid salt such as citrate, oxalate, phthalate, fumarate, maleate, succinate, malate, acetate, formate, propionate, benzoate, trifluoroacetate, methanesulfonate, benzenesulfonate, paratoluenesulfonate, and camphorsulfonate; and the base addition salt includes an inorganic base salt such as sodium salt, potassium salt, calcium salt, magnesium salt, barium salt, and aluminium salt; and an organic base salt such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, and N,N-dibenzylethylamine. Furthermore, it includes a basic or acidic amino acid salt such as arginine, lysine, ornithine, aspartate, and glutamate.

The suitable salts of starting compounds or desired compounds, and the pharmaceutically acceptable salts are conventional non-toxic salts, which include an acid addition salt such as an organic acid salt (e.g. acetate, trifluoroacetate, maleate, fumarate, citrate, tartrate, methanesulfonate, benzenesulfonate, formate, paratoluenesulfonate, etc.) and an inorganic acid salt (e.g. hydrochloride, hydrobromide, hydroiodide, sulfate, nitrate, phosphate, etc.); a salt with an amino acid (e.g. arginine, aspartate, glutamate, etc.); a metallic salt such as an alkaline metal salt (e.g. sodium salt, potassium salt, etc.) and an alkaline-earth metal salt (e.g. calcium salt, magnesium salt, etc.); ammonium salt; and an organic base salt (e.g. trimethylamine salt, triethylamine salt, pyridine salt, picoline salt, dicyclohexylamine salt, N,N'-dibenzylethylenediamine salt, etc.); and furthermore, what a skilled person selects suitably.

If it is desirable to fix the compound of the present invention as a salt, when the compound of the present invention is obtained as a salt, it may be purified without further reaction, and when it is obtained in a free form, it may be dissolved or suspended in an appropriate organic solvent and an acid or base may be added therein to form a salt in a general manner.

The compound of the present invention or a pharmaceutically acceptable salt thereof may sometimes exist in form of solvate with water or various solvents. Such solvates are also included in the present invention.

The temperature for forming a salt is selected from the range of generally -50°C to boiling point of a solvent used herein, preferably 0°C to the boiling point, and more preferably room temperature to the boiling point. In order to enhance the optical purity, it is desirable to make the temperature raised to around boiling point of a solvent used herein. In collecting a precipitated crystal on a filter, an optional cooling can make the yield increased. The amount of an optically active acid or amine used herein is suitably about 0.5 - about 2.0 equivalents against that of the substance compound, preferably around one equivalent. If appropriate, the obtained crystal may be recrystallized in an inert solvent (for example, an alcohol solvent such as methanol, ethanol, and 2-propanol; an ether solvent such as diethyl ether; an ester solvent such as ethyl acetate; a hydrocarbon solvent such as toluene; an aprotic solvent such as acetonitrile; or a mixed solvent thereof) to obtain its highly pure salt thereof. And, if appropriate, the optically-resolved salt can be also treated with an acid or a base to obtain its free form.

The compound of formula (1) in which any one or more ¹H atoms are replaced by ²H(D) atoms is also within the scope of the present invention of formula (1).

The present invention encompasses the compound of formula (1) or a pharmaceutically acceptable salt thereof. In addition, the present invention encompasses a hydrate thereof and a solvate thereof such as ethanolate thereof. Furthermore, the present invention encompasses all tautomers, stereoisomers, and crystal forms thereof.

The present compound (1) also includes an optical isomer which is based on chiral center, an atropisomer which is based on axiality caused by intramolecular rotational hindrance or planar-chirality, other stereoisomers, tautomer, and geometric isomer, all possible isomers of which and a mixture thereof are encompassed in the present invention.

The optical isomer mixture of the present compounds can be prepared in a conventional manner. The compounds having an asymmetric structure can be prepared, for example, by using a starting material having an asymmetric center or by introducing an asymmetric structure anywhere along the process. For example, in case of optical isomers, optical isomers can be obtained by using an optically active starting material or resolving a mixture of optical isomers at an appropriate step. In case that the compound of formula (1) or its intermediate has a basic functional group, the optical resolution thereof includes, for example, diastereomer method, wherein the compound is transformed to a salt thereof by reacting with an optically active acid (for example, a monocarboxylic acid such as mandelic acid, N-benzyloxyalanine, and lactic acid; dicarboxylic acid such as tartaric acid, o-diisopropylidene-tartaric acid, and malic acid; or a sulfonic acid such as camphorsulfonic acid and bromocamphorsulfonic acid), in an inert solvent (for example, an alcohols such as methanol, ethanol, and 2-propanol; an ether solvent such as diethyl ether; an ester solvent such as ethyl acetate; a hydrocarbon solvent such as toluene; an aprotic solvent such as acetonitrile; or a mixed solvent thereof). In case that the compound of formula (1) or its intermediate has an acidic functional group such as carboxyl group, the compound can be also optically resolved after forming its salt with an optically active amine (for example, an organic amine such as 1-phenylethylamine, kinin, quinidine, cinchonidine, cinchonine, and strychnine).

The present compounds of formula (1) and their intermediates can be isolated and purified in a manner known by a skilled person. It includes, for example, extraction, partition, reprecipitation, column chromatography (e.g. silica gel column chromatography, ion exchange column chromatography, and preparative liquid chromatography), and recrystallization.

The solvent for recrystallization used herein includes, for example, an alcohols solvent such as methanol, ethanol, and 2-propanol; an ether solvent such as diethyl ether; an ester solvent such as ethyl acetate; an aromatic hydrocarbon solvent such as benzene and toluene; a ketone solvent such as acetone; a halogenated solvent such as dichloromethane and chloroform; a hydrocarbon solvent such as hexane; an aprotic solvent such as dimethylformamide and acetonitrile; water; and a mixed solvent thereof. As other methods for purification, for example, methods described in Series of Experimental Chemistry (Jikken Kagaku Kouza, edited by the Chemical Society of Japan, Maruzen) Vol. 1 can be used. And, the structural determination of the present compounds can be easily done by spectroscopic analytical method such as nuclear magnetic resonance method, infrared absorption technique, and circular dichroism spectra analysis, and mass spectrometry, considering the structure of each starting compound.

Among the starting materials and the intermediates in each preparation process mentioned above, the compounds that are not described in each process are commercially available or can be prepared by a skilled person with a commercially available material in a known manner or a similar manner thereto.

The present invention provides the above-defined compound of formula (1) or a pharmaceutically acceptable salt thereof which is useful as vaccine adjuvant, preferably vaccine adjuvant for infection vaccine.

In addition, the present invention provides a pharmaceutical composition comprising the above-defined compound of formula (1) or a pharmaceutically acceptable salt thereof in combination with a pharmaceutically acceptable diluent or carrier (hereinafter, referred to as the present pharmaceutical composition).

The present compound or a pharmaceutically acceptable salt thereof may be used as an adjuvant for maintaining or enhancing the immunostimulatory of an active ingredient having an immunostimulating activity.

Namely, the present compound or a pharmaceutically acceptable salt thereof has an activity for inducing or enhancing antigen-specific antibody, specifically antigen-specific IgG, and in more detail Th1-type antigen-specific IgG (e.g. IgG2c).

And, the present compound or a pharmaceutically acceptable salt thereof has an activity for increasing cytotoxic T-lymphocyte (CTL). Or, the present compound or a pharmaceutically acceptable salt thereof has an activity for inducing CTL in mammal or enhancing the CTL induction in mammal.

And, the present compound or a pharmaceutically acceptable salt thereof has an activity for enhancing CD4-positive (i.e., MHC class II-restricted) and/or CD8-positive (i.e., MHC Class I-restricted) T-cell.

And, the present compound or a pharmaceutically acceptable salt thereof has an activity for increasing antigen-specific T-cell.

And, the present compound or a pharmaceutically acceptable salt thereof has an activity for increasing memory T-cell, specifically, CD8-positive effector memory T-cell.

And, the present compound or a pharmaceutically acceptable salt thereof has a character that the action of increasing CTL is almost the same level as the case that the same moles of an adjuvant having phosphate structure which has TLR4 agonistic action when administered to mammal.

And, the present compound or a pharmaceutically acceptable salt thereof has an activity for activating immunocompetent cells.

The pharmaceutical composition of the present invention may comprise an antigen, said antigen includes a tumor antigen or a pathogen-derived antigen. The tumor antigen includes, for example, a tumor antigen protein and a partial peptide derived from its tumor antigen protein. And, a complex of the antigen and carrier, etc. is included in the scope of the antigen in the present invention. And, the pathogen-derived antigen includes, for example, a pathogen (such as virus and bacterium) antigen protein and a partial peptide derived from its pathogen antigen protein. And, a complex of the antigen and carrier, etc. is included in the scope of the antigen in the present invention. The complex includes an antigen (including protein and peptide, but not limited thereto) bridged to a protein which is a carrier via a linker which is well known by a skilled person, and an antigen contained in virus-like particle (VLP). Thus, the present compound or a pharmaceutically acceptable salt thereof is useful as a medicament for treating or preventing infection of virus or bacterium or cancer by using in combination with the above-mentioned antigen. And, the present compound or a pharmaceutically acceptable salt thereof is useful as an adjuvant for activating the therapeutic or preventive effect for infection of virus or bacterium or cancer by using in combination with the above-mentioned antigen.

Examples of the administration route of the pharmaceutical composition of the present invention includes parenteral administration, specifically intravascular (e.g., intravenous), subcutaneous, intradermal, intramuscular, transnasal, lymph node, and transdermal administrations.

In one embodiment, the pharmaceutical composition of the present invention may comprise the compound of formula (1) or a pharmaceutically acceptable salt thereof and a pharmaceutically acceptable diluent or carrier.

The drug formulation of the present pharmaceutical composition includes a liquid formulation.

The liquid formulation of the present invention includes an aqueous solution formulation/an aqueous suspension formulation, an oily solution formulation/an oily suspension formulation, a lipid formulation, and an emulsion formulation.

The aqueous solution formulation or the aqueous suspension formulation includes, for example, a formulation prepared by dissolving or dispersing an antigen (tumor antigen or pathogen-derived antigen), and/or the compound of formula (1) or a pharmaceutically acceptable salt thereof in water.

The oily solution formulation or the oily suspension formulation includes, for example, a formulation prepared by dissolving or dispersing an antigen (tumor antigen or pathogen-derived antigen), and/or the compound of formula (1) or a pharmaceutically acceptable salt thereof in an oily ingredient.

The lipid formulation includes, for example, a liposome formulation comprising an antigen (tumor antigen or pathogen-derived antigen), and/or the compound of formula (1) or a pharmaceutically acceptable salt thereof.

The emulsion formulation includes, for example, a formulation including an aqueous solution and an oily composition, which comprises an antigen (tumor antigen or pathogen-derived antigen), and/or the compound of formula (1) or a pharmaceutically acceptable salt thereof.

The additive used in the present aqueous solution formulation or aqueous suspension formulation includes, for example, purified water, water for injection, a buffering agent, a pH adjusting agent, a stabilizer, an isotonizing agent, a solubilizer, and a solubilizing agent.

The additive used in the present oily solution formulation or oily suspension formulation includes, for example, a buffering agent, a pH adjusting agent, a stabilizer, an isotonizing agent, animal or vegetable oil and fat, hydrocarbons, a fatty acid, fatty acid esters, a solubilizer, and a solubilizing agent.

The present emulsion formulation used herein includes oil-in-water emulsion (also refered to as O/W emulsion), water-in-oil emulsion (also refered to as W/O emulsion), water-in-oil-in-water emulsion (also refered to as W/O/W emulsion), and oil-in-water-in-oil emulsion (also refered to as O/W/O emulsion). The present emulsion formulation includes, preferably water-in-oil emulsion (W/O emulsion) and oil-in-water emulsion (O/W emulsion), and more preferably oil-in-water emulsion (O/W emulsion).

The present water-in-oil emulsion formulation can be prepared by emulsifying an aqueous phase and an oil phase in a general manner. As for the present water-in-oil emulsion formulation, an antigen (tumor antigen or pathogen-derived antigen), and/or the compound of formula (1) or a pharmaceutically acceptable salt thereof may be contained in an oil phase and/or an aqueous phase.

The present oil-in-water emulsion formulation can be prepared by emulsifying an aqueous phase and an oil phase in a general manner. As for the present oil-in-water emulsion formulation, an antigen (tumor antigen or pathogen-derived antigen), and/or the compound of formula (1) or a pharmaceutically acceptable salt thereof may be contained in an oil phase and/or an aqueous phase.

In the liposome formulation of the present invention, the liposome means a microvesicle composed of lipid multiple layers such as bilayer membrane of amphiphilic lipid molecule (lipid bilayer), which has an internal phase. The preferred lipid multiple layer is lipid bilayer.

The present liposome formulation includes amphiphilic lipid molecule. The amphiphilic lipid molecule includes, preferably one or more "phospholipid". The "phospholipid" includes, for example, phosphatidylcholine, phosphatidylglycerol, phosphatidic acid, phosphatidylethanolamine, phosphatidylserine, phosphatidylinositol, and sphingomyelin. The "phospholipid" includes, preferably phosphatidylcholine, phosphatidylglycerol, phosphatidylethanolamine, sphingomyelin, and phosphatidylserine. The "phospholipid" includes, more preferably phosphatidylcholine, sphingomyelin, phosphatidylserine, and phosphatidylglycerol.

The liposome internally-including the present compound may contain sterols. The sterols includes cholesterol, β-sitosterol, stigmasterol, campesterol, brassicasterol, ergosterol, and fucosterol, and preferably cholesterol.

The liposome internally-including the present compound may contain a pharmaceutically acceptable additive. The additive includes, for example, an inorganic acid, an inorganic acid salt, an organic acid, an organic acid salt, sugars, a buffering agent, an antioxidant, and polymers. The inorganic acid includes, for example, phosphoric acid, hydrochloric acid, and sulfuric acid. The inorganic acid salt includes, for example, disodium hydrogen phosphate, sodium chloride, ammonium sulfate, and magnesium sulfate. The organic acid includes, for example, citric acid, acetic acid, succinic acid, and tartaric acid. The organic acid salt includes, for example, sodium citrate, sodium acetate, disodium succinate, and sodium tartrate. The sugar includes, for example, glucose, sucrose, mannitol, sorbitol, and trehalose. The buffering agent includes, for example, L-arginine, L-histidine, trometamol (trishydroxymethylaminomethane, Tris), and a salt thereof. The antioxidant includes, for example, sodium sulfite, L-cysteine, sodium thioglycolate, sodium thiosulfate, ascorbic acid, and tocopherol. The polymers includes, for example, polyvinyl alcohol, polyvinylpyrrolidone, carboxy vinyl polymer, and carboxymethylcellulose sodium.

The compound of formula (1), or a pharmaceutically acceptable salt thereof, or a pharmaceutical composition of the present invention may be used in combination with further another medicament besides the above pathogen-derived antigen.

The pharmaceutical composition of the present invention may further contain other additives, and examples of such additives include surfactant, antioxidants, preservatives, and soothing agents.

The compound of formula (1) or a pharmaceutically acceptable salt thereof may be administered simultaneously with the antigenic substance (immunogen) or at any interval before or after the administration of the antigenic substance in a unit dose ranging from generally about 0.1 ng/kg to 100 mg/kg to warm-blooded animal, which provides an effective dose for vaccine adjuvant. The unit dosage form for injections generally contains, for example, 1 ng to 250 mg of the active ingredient, and preferably, used at a dose ranging from 1 ng to 50 mg/kg of the active ingredient per day. However, the daily dose may vary depending on the host to be treated, the route of administration and the severity of the disease being treated. Thus, the optimal dose can be determined by a practitioner who treats individual patient or warm-blooded animal.

The term "treatment" as used herein means alleviating some or all of the symptoms of disease, in whole or in part, or preventing or delaying the progression of disease.

The term "prevention" as used herein means primary prevention of disease (prevention of onset of disease) or secondary prevention of disease (prevention of relapse in a patient whose symptom has been alleviated or disease has been cured after the onset of the disease, prevention of recurrence). When there are infection stage and onset stage to pathogens (such as bacteria, fungi, protozoa, and viruses) in an infectious disease, "prevention" includes both prevention of pathogen infection and prevention of post-infection onset. In addition, the "prevention" used herein includes the meaning of preventing transmission of a pathogen from humans to other vector organisms.

Since the compound of the present invention or a pharmaceutically acceptable salt thereof has an immune adjuvant activity *in vitro* or *in vivo,* it is useful as a vaccine adjuvant for maintaining or enhancing the immunogenicity of tumor antigen or pathogen-derived antigen.

The compound of the present invention or a pharmaceutically acceptable salt thereof has an adjuvant activity for cellular immunity *in vitro* or *in vivo,* and thus it is useful as a vaccine adjuvant for maintaining or enhancing the immunogenicity of tumor antigen or pathogen-derived antigen.

The compound of the present invention or a pharmaceutically acceptable salt thereof can be used for maintaining or enhancing the effect of an immunostimulant for treating or preventing a disease, that is a substance inducing tumor antigen-specific immune reaction or pathogen-derived antigen-specific immune reaction.

The pharmaceutical composition comprising the compound of the present invention or a pharmaceutically acceptable salt thereof, and a substance enhancing the specific immune response for tumor or pathogen (also referred to as tumor antigen-derived antigen or pathogen-derived antigen, respectively) is also included in one embodiment of the present invention. The tumor antigen or pathogen-derived antigen includes, but not limited to, a tumor-derived antigen protein, a pathogen-derived antigen protein, a tumor-derived antigen peptide derived from said tumor-derived antigen protein, a pathogen-derived antigen peptide (partial peptide) derived from said pathogen-derived antigen protein, and a complex thereof with a carrier.

In a specific embodiment of the present invention, the present compound or a pharmaceutically acceptable salt thereof can treat or prevent infection by the administration together with a pathogen-derived antigen protein or a pathogen-derived antigen peptide for preventing infection. And, the effect of the present compound or a pharmaceutically acceptable salt thereof for treating or preventing infection can be enhanced by the administration in combination with a tumor antigen protein or a tumor antigen peptide for treating or preventing infection. The preventable infectious disease includes, for example, virus diseases such as genital wart, common wart, plantar wart, hepatitis B, hepatitis C, herpes simplex virus, molluscum contagiosum, smallpox, human immunodeficiency virus (HIV), human papilloma virus (HPV), RS virus, norovirus, cytomegalovirus (CMV), varicella zoster virus (VZV), rhinovirus, adenovirus, coronavirus, influenza, and parainfluenza; bacterial diseases such as tuberculosis, mycobacterium avium, and Hansen's disease; infections such as mycosis, chlamydia, Candida, Aspergillus, cryptococcal meningitis, Pneumocystis carini, cryptosporidiosis, histoplasmosis, toxoplasmosis, malaria, Trypanosoma infection, and leishmaniasis, but should not be limited thereto. Examples of the active ingredient of the vaccine for preventing infectious include, but not limited to, substances derived from microorganisms/pathogens including bacteria, fungi, protozoa, and viruses which cause infectious diseases, such as antigenic protein, antigen peptide (partial peptide) from said antigenic protein, polysaccharide, lipid, and a combination thereof or a combination of the substance derived from said microorganisms/pathogen and a carrier.

Examples of the viral antigenic peptide derived from the viral antigen include, but not limited to, influenza matrix protein peptide 58-66 (Jager E et al., Int. J. Cancer 67: 54 (1996)), HPV16 E7 peptide 86-93 (van Driel WJ et al., Eur. J. Cancer 35:946 (1999)), HPV E7 peptide 12-20 (Scheibenbogen C et al., J. Immunother 23: 275 (2000)), HPV16 E7 peptide 11-20 (Smith JWI et al., J. Clin. Oncol. 21: 1562 (2003)), HSV2 gD (Berman PW et al., Science 227: 1490 (1985)), CMV gB (Frey SE et al., Infect Dis. 180: 1700 (1999), Gonczol E. et al., Exp. Opin. Biol. Ther. 1: 401 (2001)), and CMV pp65 (Rosa CL et al., Blood 100: 3681 (2002), Gonczol E. et al., Exp. Opin. Biol. Ther. 1: 401 (2001)).

In a specific embodiment of the present invention, the present compound or a pharmaceutically acceptable salt thereof can treat or prevent cancer by the administration in combination with a tumor antigen protein or a tumor antigen peptide for cancer immunotherapy. And, the effect of the present compound or a pharmaceutically acceptable salt thereof for treating or preventing cancer can be enhanced by the administration in combination with a tumor antigen protein or a tumor antigen peptide for cancer immunotherapy. The cancer includes, for example, leukemia, myelodysplastic syndrome, multiple myeloma, malignant lymphoma, stomach cancer, colon cancer, lung cancer, breast cancer, germ cell cancer, liver cancer, skin cancer, bladder cancer, prostate cancer, uterine cancer, cervical cancer, ovarian cancer, brain tumor, bone cancer, pancreatic cancer, head and neck cancer, skin or intraorbital malignant melanoma, rectal cancer, anal cancer, testicular cancer, fallopian tube carcinoma, endometrial carcinoma, uterocervical carcinoma, vaginal carcinoma, vulval carcinoma, Hodgkin's disease, non-Hodgkin's lymphoma, esophageal cancer, small intestinal cancer, endocrine system cancer, thyroid cancer, parathyroid cancer, adrenal cancer, soft tissue sarcoma, urethral cancer, penile cancer, acute myeloid leukemia, chronic myeloid leukemia, acute lymphoblastic leukemia, chronic or acute leukemia including chronic lymphocytic leukemia, children solid cancer, lymphocytic lymphoma, renal/ureter cancer, renal pelvic carcinoma, central nervous system (CNS) tumor, primary CNS lymphoma, tumor angiogenesis, spinal tumor, pontine glioma, pituitary adenoma, Kaposi's sarcoma, squamous cell carcinoma, planocellular carcinoma, T-cell lymphoma, polytypic glioblastoma, malignant melanoma, non-small-cell lung cancer, renal cell cancer, and asbestos-induced cancer. The treatment or prevention of cancer includes preventing metastatic disease and tumor recurrence, and preventing and treating paraneoplastic syndrome.

The carrier as used herein is a substance, such as protein and lipid, to which an antigenic protein or an antigenic peptide is bound chemically and/or physically, and the examples include, but not limited to, CRM 197 (Vaccine. 2013 Oct 1; 31(42):4827-33), KLH (Cancer Immunol Immunother. 2003 Oct; 52(10):608-16), virus-like particles (PLoS ONE 5(3): e9809) and liposomes (J Liposome Res. 2004; 14(3-4):175-89).

The antigenic protein may be prepared by cloning cDNA which encodes the antigenic protein, and expressing it in a host cell, according to a textbook such as Molecular Cloning 2nd ed., Cold Spring Harbor Laboratory Press (1989).

The synthesis of the antigenic peptide can be carried out according to a method generally used in peptide chemistry, for example, as described in literatures (Peptide Synthesis, Interscience, New York, 1966; The Proteins, Vol. 2, Academic Press Inc., New York, 1976).

In an embodiment, the present invention further provides a kit comprising:
a) a compound of the formula (1) or a pharmaceutically acceptable salt thereof, or a pharmaceutical composition comprising a compound of the formula (1) or a pharmaceutically acceptable salt thereof; and
b) a pharmaceutical composition comprising a tumor-derived antigen or a pathogen-derived antigen.

In an embodiment, the present invention further provides a kit comprising:
a) a compound of the formula (1) or a pharmaceutically acceptable salt thereof, or a pharmaceutical composition comprising a compound of the formula (1) or a pharmaceutically acceptable salt thereof; and
b) a pharmaceutical composition comprising a pathogen-derived antigen.

The antigen is not limited so long as it is an antigen that may be used as an active ingredient of vaccines, which includes antigenic proteins as mentioned above, antigenic peptides (partial peptides) derived from such antigenic proteins, and a complex thereof with a carrier.

In one embodiment of the present invention, the present invention provides use of a compound of the formula (1) or a pharmaceutically acceptable salt thereof in the preparation of a vaccine adjuvant.

Further in one embodiment of the present invention, the present invention provides use of a compound of the formula (1) or a pharmaceutically acceptable salt thereof as a vaccine adjuvant in the preparation of a vaccine for treating infection.

Further, one embodiment of the present invention provides a method for the treatment or prevention of cancer or infection, or the prevention of the progress thereof, comprising a step of administering a compound of the formula (I) as defined above, or a pharmaceutically acceptable salt thereof, together with a tumor-derived antigen or a pathogen-derived antigen, to a patient.

One embodiment of the present invention provides a method for the treatment or prevention of infection, or the prevention of the progress thereof, comprising a step of administering a compound of the formula (I) as defined above, or a pharmaceutically acceptable salt thereof, together with a pathogen-derived antigen, to a patient.

### EXAMPLES

The present invention will be further described with reference to the following examples which should not be regarded as limiting in any respect.

Troc: 2,2,2-trichloroethoxycarbonyl group
TBS: tert-butyldimethylsilyl group
Bn: benzyl group
Fmoc: 9-fluorenylmethyloxycarbonyl
DBU: diazabicycloundecene
Boc: tert-butoxycarbonyl
Alko: p-alkoxybenzyl alcohol
PEG: polyethylene glycol
tBu: tert-butyl
HBTU: O-(benzotriazol-1-yl)-N,N,N',N'-tetramethyluronium hexafluorophosphate
DIPEA: N,N-diisopropylethylamine
DMF: N,N-dimethylformamide
TFA: trifluoroacetic acid
TIS: triisopropylsilane
THF: tetrahydrofuran
TBDPS: tert-butyldiphenylsilyl group

The analysis conditions of high performance liquid chromatograph-mass spectrometer (LCMS) are shown below.

### LCMS Condition A

| | |
|---|---|
| MS detector: LCMS-IT-TOF | |
| HPLC: Shimadzu Nexera X2 LC 30AD | |
| Column: Kinetex 1.7 µ C18 100A New column 50 × 2.1 mm | |
| Flow rate: 1.2 ml/min | |
| Wave length: 254/220 nm | |
| Mobile phase: | A: 0.1 % formic acid/water |
| | B: acetonitrile |

### Time program:

| Step | Time (min) |
|---|---|
| 1 | 0.01-1.40 A:B = 90:10 - 5:95 |
| 2 | 1.40-1.60 A:B = 5:95 |
| 3 | 1.61-2.00 A:B = 99:1 |

### LCMS Condition B

| | |
|---|---|
| MS detector: ACQUITY^{™} SQ detector (Waters) | |
| HPLC: ACQUITY^{™} system | |
| Column: Waters ACQUITY^{™} UPLC BEH C18 (1.7 pm, 2.1 mm × mm) | 30 |
| Flow rate: 0.8 ml/min | |
| Wave length: 254/220 nm | |
| Mobile phase: | A: 0.06 % formic acid/acetonitrile |
| | B: 0.06 % formic acid/water |
| Time program: 0.0-1.30 A:B = 2:98 - 96:4 | |
| Column temperature: 25°C | |

### LCMS Condition C

| | |
|---|---|
| MS detector: LCMS-IT-TOF | |
| HPLC: Shimadzu Nexera X2 LC 30AD | |
| Column: none | |
| Flow rate: 1.2 ml/min | |
| Wave length: 254/220 nm | |
| Mobile phase: | A: 0.1 % formic acid/water |
| | B: acetonitrile |

### Time program:

| Step | Time (min) |
|---|---|
| 1 | 0.01-1.40 A:B = 90:10 - 5:95 |
| 2 | 1.40-1.60 A:B = 5:95 |
| 3 | 1.61-2.00 A:B = 99:1 |

### Reference example 1

### 2-(4-Bromophenyl)-2-oxoethyl (3R)-3-[(9-phenylnonanoyl)oxy]tetradecanoate

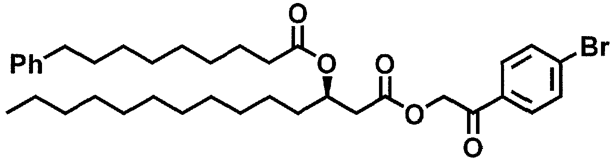

To a solution of 9-phenylnonanoic acid (398 mg) in chloroform (5 mL) were added oxalyl dichloride (0.15 mL) and DMF (1 drop), and the solution was stirred at room temperature for 3 hours. Then, 2-(4-bromophenyl)-2-oxoethyl (3R)-3-hydroxytetradecanoate (500 mg) which is a known compound and pyridine (2 mL) were added thereto, and the solution was stirred at room temperature for 18 hours. The reaction solution was extracted with water and ethyl acetate, and the organic layer was dried over anhydrous magnesium sulfate and concentrated. The obtained residue was purified by silica gel column chromatography (eluting solvent; hexane:ethyl acetate) to give the titled compound (676 mg).
¹H-NMR (400 MHz, CDCl₃): 7.73 (2H, dt, J = 9.0, 2.1 Hz), 7.60 (2H, dt, J = 9.0, 2.1 Hz), 7.27-7.22 (2H, m), 7.17-7.12 (3H, m), 5.29-5.23 (3H, m), 2.77-2.66 (2H, m), 2.57 (2H, t, J = 7.9 Hz), 2.28 (2H, t, J = 7.9 Hz), 1.70-1.50 (6H, m), 1.35-1.20 (26H, m), 0.86 (3H, dd, J = 7.9, 5.5 Hz) .

### Reference example 2

### (3R)-3-[(9-Phenylnonanoyl)oxy]tetradecanoic acid

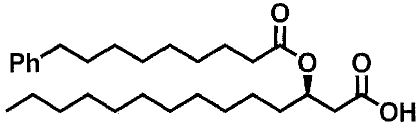

To a solution of Reference example 1 (676 mg) in acetic acid (15 mL) was added zinc powder (672 mg), and the mixture was stirred at 60°C for 4 hours. The reaction mixture was filtered with Celite, and the filtrate was concentrated. The obtained residue was purified by silica gel column chromatography (eluting solvent; chloroform:methanol) to give the titled compound (451 mg).
¹H-NMR (400 MHz, CDCl₃): 7.28-7.23 (2H, m), 7.16-7.11 (3H, m), 5.22-5.15 (1H, m), 2.64-2.52 (4H, m), 2.25 (2H, t, J = 7.6 Hz), 1.60-1.50 (6H, m), 1.32-1.20 (26H, m), 0.86 (3H, t, J = 7.0 Hz).

### Reference example 3

### (2R)-3-(Benzyloxy)-2-{[(3R)-3-(decanoyloxy)tetradecanoyl]amino}-3-oxopropyl 6-O-[tert-butyl(dimethyl)silyl]-2-{[(3R)-3-(decanoyloxy)tetradecanoyl]amino}-2-deoxy-3-O-{(3R)-3-[(9-phenylnonanoyl)oxy]tetradecanoyl}hexopyranoside

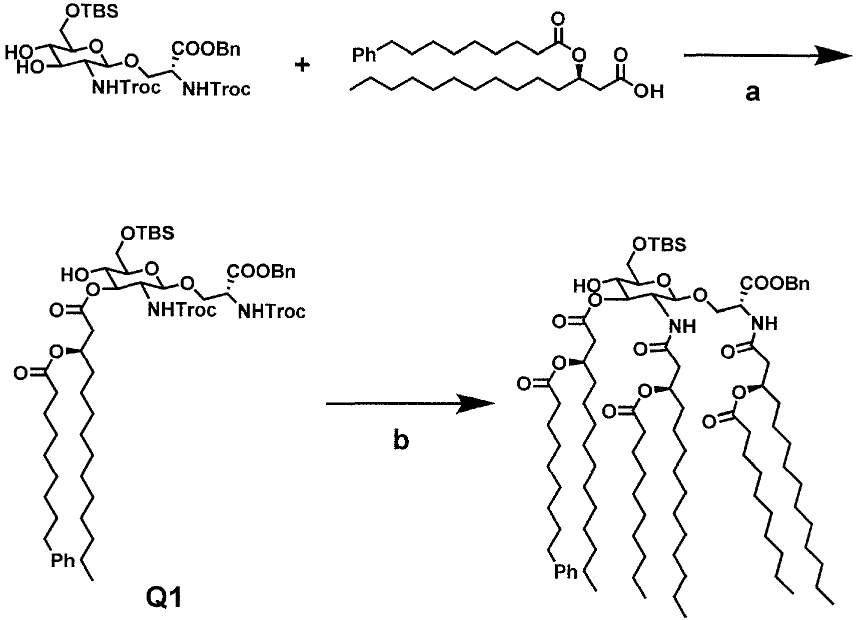

### a) Preparation of (2R)-3-(benzyloxy)-2-({[(9H-fluoren-9-yl)methoxy]carbonyl}amino)-3-oxopropyl 6-O-[tert-butyl(dimethyl)silyl]-3-O-[(3R)-3-(decanoyloxy)tetradecanoyl]-2-deoxy-2-{[(2,2,2-trichloroethoxy)carbonyl]amino}hexopyranoside (Compound Q1)

Benzyl (2*R*)-3-{[6-({[*tert-*butyl(dimethyl)silyl]oxy}methyl)-4,5-dihydroxy-3-{[(2,2,2-trichloroethoxy)carbonyl]amino}oxan-2-yl]oxy}-2-{[(2,2,2-trichloroethoxy)carbonyl]amino}propanoate (464 mg), Reference example 2 (853 mg), and 4-pyrrolidinopyridine (14 mg) were dissolved in dichloromethane (40 mL), and then 1-[3-(dimethylamino)propyl]-3-ethylcarbodiimide methiodide (550 mg) was added to the solution at 0°C. The reaction solution was stirred at room temperature for 18 hours, and then extracted with water/chloroform. The organic layer was dried over anhydrous magnesium sulfate and concentrated. The obtained residue was purified by silica gel column chromatography (eluting solvent; hexane:ethyl acetate) to give the titled Compound Q1 (1.62 g).
¹H-NMR (400 MHz, CDCl₃): 7.30 (5H, m), 7.24-7.15 (2H, m), 7.12-7.06 (3H, m), 5.95 (1H, d, J = 9.8 Hz), 5.26 (1H, d, J = 12.2 Hz), 5.12-5.02 (1H, m), 4.97 (1H, d, J = 11.7 Hz), 4.84-4.60 (5H, m), 4.50-4.40 (2H, m), 4.00-3.87 (2H, m), 3.78 (2H, qd, J = 10.8, 4.6 Hz), 3.60-3.36 (3H, m), 3.20-3.10 (1H, m), 2.57-2.42 (5H, m), 2.20 (2H, t, J = 7.6 Hz), 1.56-1.40 (6H, m), 1.39-1.20 (26H, m), 0.83-0.75 (12H, m), 0.00 (6H, s).

### b) Preparation of Reference example 3

Compound Q1 (1.62 g) was dissolved in acetic acid (18 mL), and zinc powder (2.79 g) was added to the solution. The reaction mixture was stirred at room temperature for 2 hours, and filtered with Celite. The filtrate was neutralized with sodium hydrogen carbonate and extracted with chloroform. The organic layer was dried over anhydrous magnesium sulfate, filtered, and concentrated. The obtained residue (1.15 g) and (3R)-3-(decanoyloxy)tetradecanoic acid (1.10 g) were dissolved in dichloromethane (70 mL), and 1-ethoxycarbonyl-2-ethoxy-1,2-dihydroquinoline (746 mg) was added to the solution. The reaction solution was stirred at room temperature for 16 hours. The reaction solution was purified by silica gel column chromatography (eluting solvent; hexane:ethyl acetate) to give the titled compound (1.38 g).
¹H-NMR (400 MHz, CDCl₃): 7.35-7.32 (5H, m), 7.21-7.17 (2H, m), 7.12-7.07 (3H, m), 6.65 (1H, d, J = 8.5 Hz), 5.44 (1H, d, J = 8.5 Hz), 5.22-4.94 (5H, m), 4.69-4.61 (2H, m), 3.91-3.73 (5H, m), 3.65 (1H, q, J = 9.5 Hz), 3.49 (1H, t, J = 11.6 Hz), 3.39 (1H, d, J = 2.4 Hz), 3.11-3.06 (1H, m), 2.54-2.16 (14H, m), 1.51 (14H, s), 1.20 (86H, m), 0.82-0.78 (24H, m), 0.00 (6H, s).

### Reference example 4

### (2R)-3-(Benzyloxy)-3-oxo-2-({(3R)-3-[(9-phenylnonanoyl)oxy]tetradecanoyl}amino)propyl 6-O-[tert-butyl(dimethyl)silyl]-3-O-[(3R)-3-(decanoyloxy)tetradecanoyl]-2-{[(3R)-3-(decanoyloxy)tetradecanoyl]amino}-2-deoxyhexopyranoside

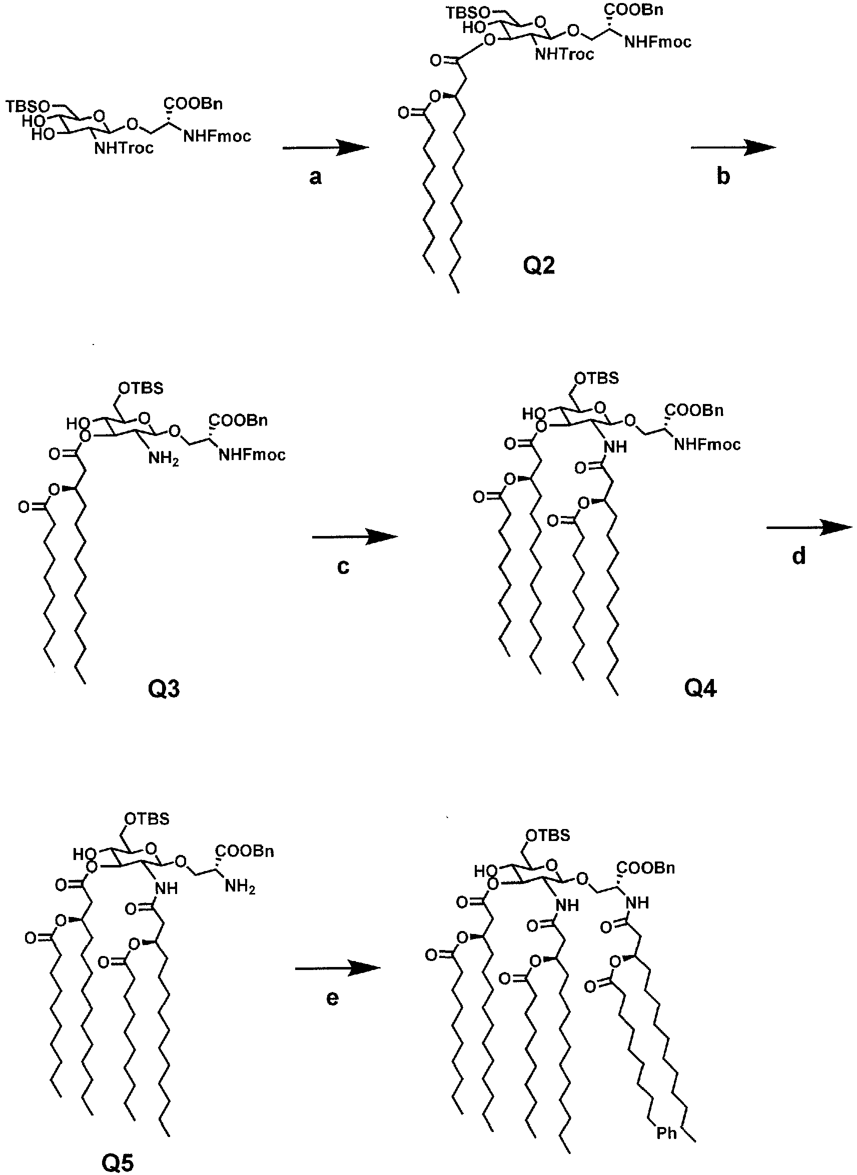

### a) Preparation of (2R)-3-(benzyloxy)-2-({[(9H-fluoren-9-yl)methoxy]carbonyl}amino)-3-oxopropyl 6-O-[tert-butyl(dimethyl)silyl]-3-O-[(3R)-3-(decanoyloxy)tetradecanoyl]-2-deoxy-2-{[(2,2,2-trichloroethoxy)carbonyl]amino}hexopyranoside (Compound Q2)

The titled compound Q2 was prepared in a similar reaction and treatment to Step a) described in Reference example 3.
LCMS (Condition A): 1271.5 [M+Na⁺], 1.26 min

### b) Preparation of (2R)-3-(benzyloxy)-2-({[(9H-fluoren-9-yl)methoxy]carbonyl}amino)-3-oxopropyl 2-amino-6-O-[tert-butyl(dimethyl)silyl]-3-O-[(3R)-3-(decanoyloxy)tetradecanoyl]-2-deoxyhexopyranoside (Compound Q3)

Compound Q2 (220 mg) was dissolved in acetic acid (2 mL), and zinc powder (346 mg) was added to the solution. The reaction mixture was stirred at room temperature for 2.5 hours, and filtered with Celite. The filtrate was extracted with water/ethyl acetate. The organic layer was dried over anhydrous magnesium sulfate and concentrated. The obtained residue was purified by silica gel column chromatography (eluting solvent; hexane:ethyl acetate) to give the titled Compound Q3 (114 mg).
1H-NMR (CDCl3) δ: 7.73 (2H, d, J = 7.9 Hz), 7.57 (2H, d, J = 6.7 Hz), 7.38-7.25 (9H, m), 5.88 (1H, d, J = 8.5 Hz), 5.22-5.08 (3H, m), 4.73 (1H, t, J = 9.4 Hz), 4.54 (1H, d, J = 8.5 Hz), 4.40 (1H, dd, J = 10.4, 6.7 Hz), 4.29 (1H, dd, J = 10.7, 7.6 Hz), 4.20 (1H, t, J = 7.3 Hz), 4.10-3.95 (3H, m), 3.90-3.74 (2H, m), 3.55 (1H, t, J = 9.1 Hz), 3.40-3.20 (2H, m), 2.71-2.58 (3H, m), 2.25 (2H, t, J = 7.3 Hz), 1.65-1.30 (6H, m), 1.30-1.10 (30H, m), 0.86-0.80 (15H, m), 0.01 (6H, d, J = 3.0 Hz).

### c) Preparation of (2R)-3-(benzyloxy)-2-({[(9H-fluoren-9-yl)methoxy]carbonyl}amino)-3-oxopropyl 6-O-[tert-butyl(dimethyl)silyl]-3-O-[(3R)-3-(decanoyloxy)tetradecanoyl]-2-{[(3R)-3-(decanoyloxy)tetradecanoyl]amino}-2-deoxyhexopyranoside (Compound Q4)

Compound Q3 (114 mg) and (3R)-3-(decanoyloxy)tetradecanoic acid (46.6 mg) were dissolved in dichloromethane (3 mL), and 1-ethoxycarbonyl-2-ethoxy-1,2-dihydroquinoline (31.5 mg) was added to the solution. The reaction solution was stirred at room temperature for 16 hours. The reaction solution was purified by silica gel column chromatography (eluting solvent; hexane:ethyl acetate) to give the titled Compound Q4 (107 mg).
¹H-NMR (400 MHz, CDCl₃): 7.70 (2H, d, J = 7.9 Hz), 7.59 (2H, d, J = 7.3 Hz), 7.36-7.21 (9H, m), 5.94 (1H, d, J = 9.1 Hz), 5.49 (1H, d, J = 8.5 Hz), 5.25 (1H, d, J = 12.2 Hz), 5.15-5.01 (3H, m), 4.74 (1H, t, J = 10.1 Hz), 4.43 (1H, d, J = 9.1 Hz), 4.33 (1H, dd, J = 10.4, 7.3 Hz), 4.25 (1H, t, J = 8.8 Hz), 4.18 (1H, t, J = 7.3 Hz), 4.03-3.92 (3H, m), 3.83-3.69 (3H, m), 3.53 (1H, t, J = 9.1 Hz), 3.41 (1H, d, J = 2.4 Hz), 3.18-3.10 (1H, m), 2.57-2.37 (3H, m), 2.26-2.21 (5H, m), 1.60-1.40 (8H, m), 1.30-1.10 (60H, m), 0.86-0.79 (21H, m), 0.00 (6H, d, J = 2.4 Hz).

### d) Preparation of (2R)-2-amino-3-(benzyloxy)-3-oxopropyl 6-O-[tert-butyl(dimethyl)silyl]-3-O-[(3R)-3-(decanoyloxy)tetradecanoyl]-2-{ [(3R)-3-(decanoyloxy)tetradecanoyl]amino}-2-deoxyhexopyranoside (Compound Q5)

To a solution of Compound Q4 (107 mg) in dichloromethane (3 mL) was added a solution of DBU (4.48 mg) in dichloromethane (0.1 mL), and the solution was stirred at room temperature for 30 minutes. Then, the reaction solution was directly loaded on silica gel and purified by silica gel column chromatography (eluting solvent; chloroform:methanol) to give Compound Q5 (89 mg).
¹H-NMR (400 MHz, CDCl₃): 7.34-7.27 (5H, m), 5.68 (1H, d, J = 9.2 Hz), 5.16 (1H, d, J = 12.2 Hz), 5.10-5.01 (3H, m), 4.75 (1H, dd, J = 11.0, 9.2 Hz), 4.16 (1H, d, J = 7.9 Hz), 3.98 (1H, dd, J = 9.8, 3.1 Hz), 3.82-3.38 (7H, m), 3.20-3.15 (1H, m), 2.54-2.34 (3H, m), 2.24-2.18 (5H, m), 1.60-1.40 (8H, m), 1.28-1.10 (60H, m), 0.83-0.76 (21H, m), 0.00 (6H, d, J = 2.4 Hz).

### e) Preparation of Reference example 4

To a solution of Reference example Q5 (89 mg) and Reference example 2 (36.5 mg) in dichloromethane (3 mL) was added 1-ethoxycarbonyl-2-ethoxy-1,2-dihydroquinoline (21.4 mg), and the reaction solution was stirred at room temperature for 18 hours. The reaction solution was concentrated and the residue was purified by silica gel column chromatography (eluting solvent; hexane:ethyl acetate) to give Reference example 4 (97 mg).
¹H-NMR (400 MHz, CDCl₃): 7.37-7.17 (7H, m), 7.12-7.07 (3H, m), 6.68 (1H, d, J = 8.5 Hz), 5.45 (1H, d, J = 8.5 Hz), 5.22-4.94 (5H, m), 4.70-4.60 (2H, m), 3.90-3.70 (5H, m), 3.65 (1H, td, J = 9.3, 7.3 Hz), 3.49 (1H, t, J = 9.5 Hz), 3.41 (1H, d, J = 2.4 Hz), 3.12-3.07 (1H, m), 2.53-2.36 (7H, m), 2.24-2.15 (7H, m), 1.60-1.40 (14H, m), 1.30-1.10 (86H, d, J = 9.8 Hz), 0.82-0.78 (24H, m), 0.00 (6H, s).

### Reference examples 5 - 8

Each compound shown in Table 1 was prepared from each corresponding starting compound in a similar reaction and treatment to the method described in Reference example 3 or 4.

| Table 1 | | |
|---|---|---|
| Reference example | Structure | ¹H-NMR (400 MHz, CDCl₃) |
| 5 | 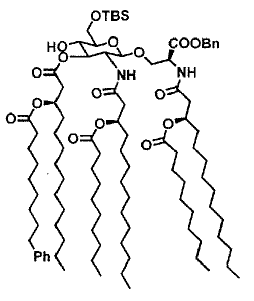 | 7.30-7.18 (7H, m), 7.11-7.08 (3H, m), 6.98 (1H, d, J = 7.9 Hz), 6.20 (1H, d, J = 7.9 Hz), 5.09-5.02 (5H, m), 4.90 (1H, t, J = 9.8 Hz), 4.67-4.60 (1H, m), 4.53 (1H, d, J = 8.5 Hz), 4.14 (1H, dd, J = 10.7, 3.4 Hz), 3.85-3.56 (5H, m), 3.41-3.33 (1H, m), 3.29-3.20 (1H, m), 2.62-2.19 (14H, m), 1.49 (14H, brs), 1.25 (86H, m), 0.82-0.78 (24H, m), 0.00 (6H, s). |
| 6 | 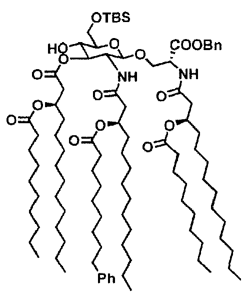 | 7.40-7.31 (7H, m), 7.10-7.08 (3H, m), 6.70-6.67 (1H, m), 5.45 (1H, d, J = 8.4 Hz), 5.21-5.16 (3H, m), 5.10-4.95 (4H, m), 4.69-4.62 (2H, m), 3.91-3.83 (4H, m), 3.80-3.61 (4H, m), 3.54-3.46 (2H, m), 3.12-3.07 (1H, m), 2.56-2.36 (14H, m), 1.50 (14H, brs), 1.18 (86H, m), 0.86-0.79 (24H, m), 0.00 (6H, s). |
| 7 | 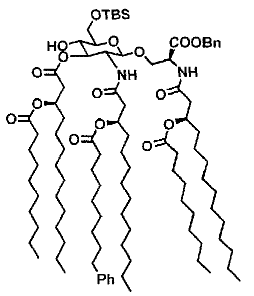 | 7.36-7.32 (7H, m), 7.10-7.08 (3H, m), 6.70-6.67 (1H, m), 5.49 (1H, d, J = 8.0 Hz), 5.21-4.95 (5H, m), 4.79-4.62 (2H, m), 3.92-3.61 (6H, m), 3.52-3.43 (2H, m), 3.12-3.08 (1H, m), 2.54-2.36 (8H, m), 2.23-2.10 (6H, m), 1.52 (14H, brs), 1.18 (86H, m), 0.85-0.79 (24H, m), 0.00 (6H, s). |
| 8 | 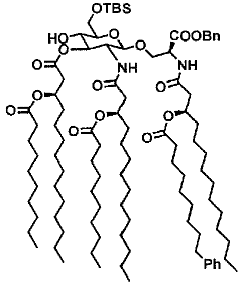 | 7.30-7.18 (7H, m), 7.11-7.08 (3H, m), 6.96 (1H, t, J = 11.0 Hz), 6.18 (1H, d, J = 7.9 Hz), 5.12-5.00 (5H, m), 4.90 (1H, dd, J = 11.0, 9.1 Hz), 4.66-4.63 (1H, m), 4.53 (1H, d, J = 7.9 Hz), 4.14 (1H, dd, J = 11.0, 3.0 Hz), 3.85-3.56 (5H, m), 3.40 (1H, s), 3.29-3.24 (1H, m), 2.61-2.19 (14H, m), 1.60-1.42 (14H, m), 1.30-1.10 (86H, m), 0.84-0.78 (24H, m), 0.00 (6H, s). |

### Example 1

### (2R)-2-{[(3R)-3-(Decanoyloxy)tetradecanoyl]amino}-3-{[3-{[(3R)-3-(decanoyloxy)tetradecanoyl]amino}-5-hydroxy-6-(hydroxymethyl)-4-({(3R)-3-[(9-phenylnonanoyl)oxy]tetradecanoyl}oxy)oxan-2-yl]oxy}propanoic acid

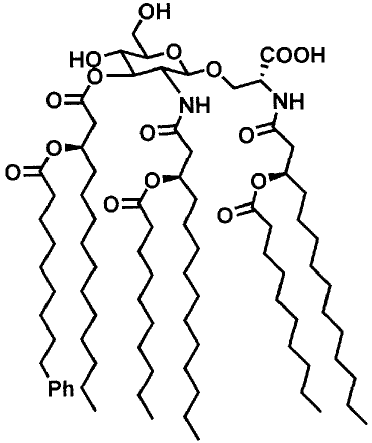

Reference example 3 (33 mg) and 10 % palladium carbon (25.9 mg) were added to THF (2 mL), and the mixture was stirred at room temperature at one atm under hydrogen atmosphere for 8 hours. The reaction mixture was filtered with Celite, and the filtrate was concentrated. To the residue were added THF (1 mL) and TFA (0.1 mL), and the mixture was stirred at room temperature for 3 hours. After the reaction was completed, the reaction mixture was neutralized with sodium hydrogen carbonate and extracted with chloroform. The organic layer was dried over anhydrous magnesium sulfate, and concentrated. The obtained residue was purified by silica gel column chromatography (eluting solvent; chloroform:methanol) to give the titled compound (5.1 mg).
¹H-NMR (400 MHz, CDCl₃/CD₃OD(30:1)): 7.19 (2H, d, J = 7.6 Hz), 7.11-7.08 (3 H, m), 5.18-5.14 (1H, m), 5.04 (2H, s), 4.83 (1H, t, J = 9.1 Hz), 4.51 (1H, s), 4.32 (1H, d, J = 8.5 Hz), 3.95-3.60 (4H, m), 3.49 (1H, t, J = 9.4 Hz), 3.35-3.26 (2H, m), 2.54-2.37 (6H, m), 2.26-2.18 (8H, m), 1.51 (14H, s), 1.18 (86H, s), 0.81 (15H, t, J = 6.7 Hz).

### Examples 2 - 5

Each compound shown in Table 2 was prepared from each corresponding starting compound in a similar reaction and treatment to the method described in Example 1.

| Table 2 | | | | | |
|---|---|---|---|---|---|
| 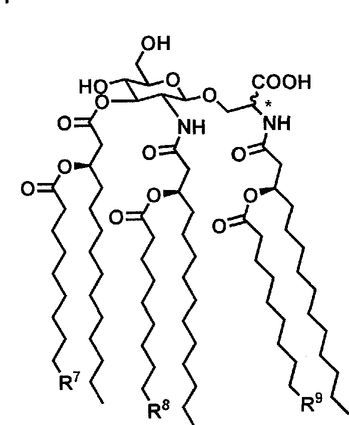 | | | | | |
| Example | R⁷ | R⁸ | R⁹ | Stereochemistry of * | ¹H-NMR (400 MHz, CDCl₃/CD₃OD (30:1)) |
| 2 | Ph | Me | Me | S | 7.19 (2H, d, J = 6.7 Hz), 7.11-7.08 (3 H, m), 5.19-5.00 (3H, m), 4.83 (1H, t, J = 9.8 Hz), 4.37 (2H, d, J = 7.9 Hz), 4.07 (1H, d, J = 9.1 Hz), 3.86-3.60 (4H, m), 3.47 (1H, t, J = 9.4 Hz), 3.35-3.26 (2H, m), 2.57-2.36 (6H, m), 1.53 (14H, s), 1.19 (86H, s), 0.81 (15H, t, J = 7.2 Hz) . |
| 3 | Me | Ph | Me | R | 7.18 (2H, d, J = 7.2 Hz), 7.10-7.08 (3H, m), 5.13-5.06 (3H, m), 4.89 (1H, t, J = 8.0 Hz), 4.44-4.35 (2H, d, J = 3.6 Hz), 4.29 (1H, s), 3.93 (1H, br), 3.86-3.62 (4H, m), 3.38-3.35 (2H, m), 2.54-2.44 (6H, m), 2.33-2.19 (8H, m), 1.57-1.44 (14H, m), 1.30-1.12 (86H, m), 0.81 (15H, t, J = 6.7 Hz) . |
| 4 | Me | Me | Ph | R | 7.18 (2H, d, J = 7.3 Hz), 7.11-7.07 (3H, m), 5.20-5.00 (3H, m), 4.86 (1H, t, J = 9.8 Hz), 4.42-4.35 (2H, m), 3.93 (1H, d, J = 7.9 Hz), 3.84-3.64 (4H, m), 3.46 (1H, t, J = 9.5 Hz), 3.34-3.25 (2H, m), 2.55-2.42 (6H, m), 2.30-2.10 (8H, m), 1.58-1.45 (14H, m), 1.28-1.15 (86H, m), 0.81 (15H, t, J = 6.7 Hz). |
| 5 | Me | Me | Ph | S | 7.18 (2H, d, J = 7.3 Hz), 7.11-7.07 (3H, m), 5.14-5.02 (3H, m), 4.85 (1H, t, J = 10.1 Hz), 4.38 (1H, d, J = 8.5 Hz), 4.30 (1H, s), 4.08 (1H, d, J = 11.0 Hz), 3.83-3.64 (4H, m), 3.44 (1H, t, J = 9.5 Hz), 3.34-3.28 (3H, m), 2.50-2.10 (14H, m), 1.60-1.40 (14H, m), 1.28-1.12 (86H, m), 0.81 (15H, t, J = 7.0 Hz). |

### Reference example 9

### (2R)-2-{[(3R)-3-(Decanoyloxy)tetradecanoyl]amino}-3-{[3-{[(3R)-3-(decanoyloxy)tetradecanoyl]amino}-6-(hydroxymethyl)-4-({(3R)-3-[(9-phenylnonanoyl)oxy]tetradecanoyl}oxy)-5-(phosphonooxy)oxan-2-yl]oxy}propanoic acid

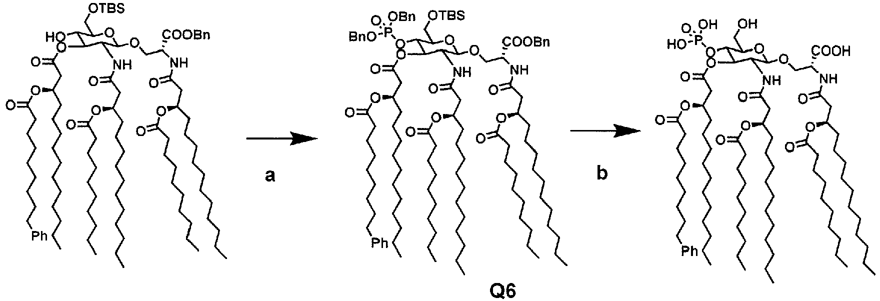

### a) Preparation of (2R)-3-(benzyloxy)-2-{[(3R)-3-(decanoyloxy)tetradecanoyl]amino}-3-oxopropyl 4-O-[bis(benzyloxy)phosphoryl]-6-O-[tert-butyl(dimethyl)silyl]-2-{[(3R)-3-(decanoyloxy)tetradecanoyl]amino}-2-deoxy-3-O-{(3R)-3-[(9-phenylnonanoyl)oxy]tetradecanoyl}hexopyranoside (Compound Q6)

To a solution of Reference example 3 (400 mg) in dichloromethane (8 mL) were added dibenzyl N,N-diisopropylphosphoramidite (231 mg) and 4,5-dicyanoimidazole (79 mg), and the solution was stirred at room temperature for 18 hours. Then, 3-chloroperoxybenzoic acid (190 mg) was added to the reaction solution, and the mixture was stirred at 0°C for an hour. Aqueous sodium hydrogen carbonate was added to the reaction mixture, and the mixture was stirred at room temperature for 15 minutes and then extracted with chloroform. The organic layer was dried over anhydrous magnesium sulfate and concentrated. The obtained residue was purified by silica gel column chromatography (eluting solvent; hexane:ethyl acetate) to give Compound Q6 (365 mg).
¹H-NMR (400 MHz, CDCl₃): 7.43-7.24 (17H, m), 7.19-7.13 (3H, m), 6.79 (1H, d, J = 8.2 Hz), 5.81 (1H, d, J = 8.2 Hz), 5.29-5.05 (6H, m), 4.96 (4H, dd, J = 18.3, 7.8 Hz), 4.75-4.68 (1H, m), 4.31-4.25 (2H, m), 4.00-3.85 (3H, m), 3.68 (1H, dd, J = 11.4, 5.5 Hz), 3.55 (1H, dt, J = 14.5, 5.6 Hz), 3.31-3.26 (1H, m), 2.61-2.18 (14H, m), 1.70-1.50 (14H, m), 1.35-1.18 (86H, dd, J = 34.3, 27.4 Hz), 0.88 (15H, td, J = 6.7, 2.4 Hz), 0.85 (9H, s), 0.00 (6H, d, J = 5.5 Hz).

### b) Preparation of Reference example 9

To a solution of Compound Q6 (365 mg) in THF (20 mL) were added 10 % palladium carbon (248 mg) and 2 mol/L hydrochloric acid (0.5 mL), and the mixture was stirred at room temperature under hydrogen atmosphere (4 atm) for 8 hours. The reaction mixture was filtered with Celite, and the filtrate was concentrated. The obtained residue was purified by silica gel column chromatography (eluting solvent; chloroform:methanol (the methanol contained 2.5 % water and 2.5 % triethylamine)) to give the titled compound (140 mg).
¹H-NMR (400 MHz, CDCl₃/CD₃OD(30:1)): 7.19 (2H, d, J = 7.3 Hz), 7.11-7.07 (3H, m), 5.22-4.99 (4H, m), 4.61-4.52 (1H, m), 4.42-4.32 (1H, m), 4.21-4.10 (1H, m), 3.95-3.80 (2H, m), 3.80-3.60 (2H, m), 3.34-3.20 (2H, m), 2.97 (4H, q, J = 7.3 Hz), 2.60-2.30 (6H, m), 2.30-2.10 (8H, m), 1.60-1.40 (14H, m), 1.30-1.10 (86H, m), 0.80 (15H, t, J = 6.7 Hz).

### Reference examples 10 - 14

Each compound shown in Table 3 was prepared from each corresponding starting compound in a similar reaction and treatment to the method described in Reference example 9.

| Table 3 | | | | | |
|---|---|---|---|---|---|
| 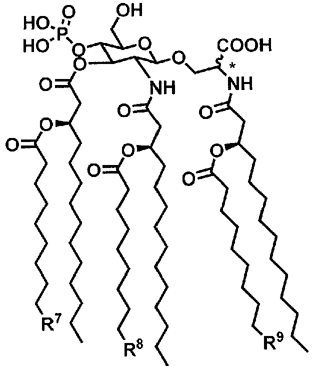 | | | | | |
| Reference example | R⁷ | R⁸ | R⁹ | Stereochemistry of * | ¹H-NMR (400 MHz, CDCl₃/CD₃OD (30:1)) |
| 10 | Ph | Me | Me | S | 7.18 (2H, d, J = 6.7 Hz), 7.11-7.07 (3H, m), 5.18-5.05 (4H, m), 4.51-4.40 (2H, m), 4.20-4.05 (2H, m), 3.78-3.60 (4H, m), 3.4-3.38 (2H, m), 2.65-2.10 (14H, m), 1.60-1.40 (14H, m), 1. 30-1.10 (86H, m), 0.81 (15H, t, J = 6.7 Hz) . |
| 11 | Me | Ph | Me | R | 7.18 (2H, d, J = 7.2 Hz), 7.11-7.09 (3H, m), 5.17-5.00 (4H, m), 4.47 (1H, d, J = 3.6 Hz), 4.32 (1H, s), 4.14-3.84 (4H, m), 3.68-3.57 (2H, m), 3.34-3.25 (3H, m), 2.63-2.35 (8H, m), 2.30-2.12 (6H, m) 1.60-1.40 (14H, m), 1.25-1.10 (86H, m), 0.81 (15H, t, J = 6.7 Hz). |
| 12 | Me | Ph | Me | S | 7.18 (2H, d, J = 7.2 Hz), 7.11-7.09 (3H, m), 5.27-5.13 (4H, m), 3.66-3.35 (4H, m), 3.08-3.04 (2H, m), 2.51-2.50 (3H, m), 2.20-1.99 (24H, m), 1.60-1.40 (14H, m), 1. 31-1.18 (86H, m), 0.78 (15H, t, J = 6.7 Hz). |
| 13 | Me | Me | Ph | R | 7.20-7.05 (5H, m), 5.20-4.98 (4H, m), 4.43 (1H, d, J = 8.5 Hz), 4.35 (1H, s), 4.12 (1H, q, J = 9.1 Hz), 3.92-3.80 (3H, m), 3.72-3.60 (2H, m), 3.34-3.10 (2H, m), 2.60-2.10 (14H, m), 1.58-1.40 (14H, m), 1.25-1.10 (86H, m), 0.80 (15H, t, J = 6.7 Hz). |
| 14 | Me | Me | Ph | S | 7.18 (2H, d, J = 7.3 Hz), 7.11-7.07 (3H, m), 5.10-5.02 (4H, m), 4.53 (1H, d, J = 8.5 Hz), 4.33 (1H, s), 4.15-3.90 (4H, m), 3.67-3.59 (2H, m), 3.35-3.33 (2H, m), 3.24-3.20 (1H, m), 2.60-2.15 (14H, m), 1.60-1.40 (14H, m), 1.25-1.10 (86H, m), 0.81 (15H, t, J = 6.7 Hz). |

### Reference example 15

Each compound shown in Table 4 was prepared from each corresponding starting compound in a similar reaction and treatment to the method described in Example 1.

| Table 4 | | | | | |
|---|---|---|---|---|---|
| 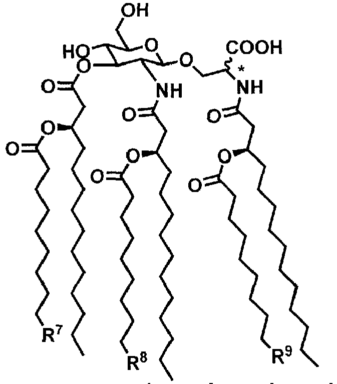 | | | | | |
| Reference example | R⁷ | R⁸ | R⁹ | Stereochemistry of * | ¹H-NMR (400 MHz, CDCl₃/CD₃OD (30:1)) |
| 15 | Me | Ph | Me | S | 7.18 (2H, d, J = 7.2 Hz), 7.10-7.08 (3H, m), 5.13-5.06 (3H, m), 4.85 (1H, t, J = 8.8 Hz), 4.41-4.11 (3H, m), 3.77-3.64 (4H, m), 3.38-3.35 (2H, m), 2.57-2.42 (6H, m), 2.28-2.11 (8H, m), 1.57-1.44 (14H, m), 1.33-1.08 (86H, m), 0.81 (15H, t, J = 6.7 Hz). |

### Test 1

### Human TLR4 reporter gene assay

HEK-Blue^{™} hTLR4 cell line (Invivogen Corporation) is a stably co-transfected cell line which expresses human TLR4, MD2, CD14, and secretory alkaline phosphatase (SEAP) reporter gene under the transcriptional regulation of an NF-κB response element. The TLR4 expression of the cell line has been already tested by RT-PCR, flow cytometry. Transfectants with stable expression were selected using the antibiotic HEK-Blue^{™} Selection. TLR signaling leads to the translocation of NF-κB and the activation of the promoter results in expression of the SEAP gene. TLR4-specific activation was assessed by determining the level of SEAP produced following incubation of the cells at 37°C for 16 - 20 hours with each compound prepared in Examples and Reference examples in the presence of 0.1 % (v/v) DMSO. The human TLR4 activity for the present compound was assessed by human TLR4 reporter gene assay, and the concentration of the compound concentration which produced half of the maximal level of SEAP induced with lipopolysaccharide (LPS) was determined as EC₅₀.

### Test 2

### Mouse TLR4 reporter gene assay

HEK-Blue^{™} mTLR4 cell line (Invivogen) is a stably co-transfected cell line which expresses mouse TLR4, MD2, CD14 and secretory SEAP reporter gene under the transcriptional regulation of an NF-κB response element. The TLR4 expression of the cell line has been already tested by RT-PCR, flow cytometry. Transfectants with stable expression were selected using the antibiotic HEK-Blue^{™} Selection. TLR signaling leads to the translocation of NF-κB and the activation of the promoter results in expression of the SEAP gene. TLR4-specific activation was assessed by determining the level of SEAP produced following incubation of the cells at 37°C for 16 - 20 hours with each compound prepared in Examples and Reference examples in the presence of 0.1 % (v/v) DMSO. The mouse TLR4 activity for the present compound was assessed by mouse TLR4 reporter gene assay, and the concentration of the compound concentration which produced half of the maximal level of SEAP induced with LPS was determined as EC₅₀.

The results of Tests 1 and 2 are shown in Tables 5 and 6.

| Table 5 | | |
|---|---|---|
| Example | human TLR4 EC₅₀ (ng/ mL) | mouse TLR4 EC₅₀ (ng/ mL) |
| 1 | 64 | 21 |
| 2 | 22 | 21 |
| 3 | 237 | 202 |
| 4 | 77 | 57 |
| 5 | 148 | 73 |

| Table 6 | | |
|---|---|---|
| Reference example | human TLR4 EC₅₀ (ng/ mL) | mouse TLR4 EC₅₀ (ng/ mL) |
| 9 | 68 | 44 |
| 10 | 20 | 20 |
| 11 | 708 | 78 |
| 12 | >1000 | >1000 |
| 13 | 53 | 21 |
| 14 | 22 | 22 |

The results in Table 5 have clarified that the example compounds of the present invention have TLR4 agonistic effect, which are almost equal or higher than those of Reference example 9 - 14 shown in Table 6 which have phosphate group.

### Preparation of liposome formulation (Examples 6 - 7, Reference examples 16 - 17)

1,2-Dimyristoyl-sn-glycero-3-phosphocholine (35.45 mg), egg yolk phosphatidylglycerol (24.45 mg), each compound shown in Table 7 (6 mg) were dissolved in t-butyl alcohol, and the solution was lyophilized. Phosphate buffered saline (3 mL) was added to the lyophilized product. The obtained solution was allowed to pass through 0.1 µm polycarbonate membrane with an extruder heated at about 65°C (Mini-Extruder, Avanti Polar Lipids) to prepare a liposome formulation. The preparation scale was optionally changed if necessary. In the liposome formulation of Reference example 17, the compound of Reference example 10 was not detected under LCMS Condition C. It was supposed that the compound of Reference example 10 was not formed to liposomes, i.e., it was trapped with the membrane of the extruder.

The above liposome was diluted 10 times with purified water, and the mean particle size, polydisperse index, and zeta potential of the diluted liposome were measured with a dynamic light scattering (ZETASIZER Nano-ZS, Malvern). And, the formulations of Examples 6 and 7 and Reference example 16 were measured about the content with high-performance liquid chromatography (HPLC).

### HPLC Condition

Detector: UV (205 nm)
HPLC: Shimadzu LC 20AD
Column: XSelect CSH Phenyl-Hexyl 2.5 µm 75 × 4.6 mm
Flow rate: 0.8 ml/min
Mobile phase: A: 0.1 % trifluoroacetic acid/water
B: 2-propanol
Time program:

| Step | Time (min) | |
|---|---|---|
| 1 | 0.0-7.0 | A:B = 20: 80 |
| 2 | 7.0-7.5 | A:B = 20: 80 - 5: 95 |
| 3 | 7.5-11.0 | A:B = 5: 95 |
| 4 | 11.0-11.1 | A:B = 5: 95 - 20: 80 |
| 5 | 11.1-15.0 | A:B = 20: 80 |

The results are shown in Table 7.

| Table 7 | | | | | |
|---|---|---|---|---|---|
| Example/ Reference example | Compound | Content (mg/ mL) | mean particle size (Z-average, nm) | polydisperse index (PDI) | zeta potential (mV) |
| Example 6 | Example 1 | 2.1 | 98.4 | 0.229 | -74.7 |
| Example 7 | Example 2 | 2.2 | 84.6 | 0.212 | -71.4 |
| Reference example 16 | Reference example 9 | 2.0 | 123.0 | 0.122 | -64.8 |
| Reference example 17 | Reference example 10 | impossible to measure | | | |

### Test 3

An equal mixture of ovalbumin (OVA) (2 mg/ mL) as an antigen and the formulation prepared in Example 6, Example 7, or Reference example 16 (containing 0.2 or 2 mg/mL the compound of Example 1, Example 2, or Reference example 9) was intramuscularly administered to the gastrocnemius of a 7-week-old C57BL/6 male mouse (100 µL/mouse), which was defined as the first immunization. Two weeks later, the equal mixture was intramuscularly administered to the gastrocnemius again, which was defined as the additional immunization. One week after the additional immunization, the heart blood was collected under inhalation anesthesia, and the serum was collected from the blood by centrifugation. The OVA-specific IgG2c value in each serum was measured by the following ELISA method. To 96-well plate, OVA solution (SIGMA) was added, 1 % Skim Milk (Wako) was added for blocking, the serum sample which was diluted with phosphate buffer solution was added, caprine antimouse IgG2c (Southern Bio) as the secondary antibody was added, SureBlue^{™} TMB Micrewell Peroxidase Substrate (KPL) was added, and then the product of the enzymatic reaction was quantified with a microplate reader. The results are shown in Figures 1 and 2.

Example 1, Example 2, and Reference example 9 exhibited significantly strong OVA-specific IgG2c induction, compared with the negative control group.

### Test 4

The equal mixture prepared in Test 3 of ovalbumin (OVA) (2 mg/mL) and the formulation prepared in Example 6, Example 7, or Reference example 16 (containing 0.2 or 2 mg/mL the compound of Example 1, Example 2, or Reference example 9) was administered to a mouse (100 pL/mouse). The spleen cell of the mouse was prepared, OVA and Brefeldin A (eBioscience) were added the cell, and the cell was cultured overnight. The collected cell was stained with APC-labeled antimouse CD3e antibody (Invitrogen), PerCP-labeled antimouse CD4 antibody (BioLegend), and Fixable Viability Dye eFluor^{™} 520 (invitorgen), and fixed with Fixation/Permeabilization buffer (Invitrogen). The cell was treated with Permeabilization buffer (Invitrogen), and then stained with antibody cocktail BV421-labeled anti-IFN-γ antibody (BioLegend), PE-Cy7-labeled anti-IL-2 antibody (eBioscience), and PE-labeled TNF-α (BioLegend). The data were taken and analyzed with FACS Cant II (BD Biosciences) and FLOWJO software (TreeStar). The results are shown in Figures 3 and 4.

Separately, the spleen cell was stained with V450-labled antimouse CD3e antibody (invitrogen), Alexa Fluor^{™} 647-labeled antimouse CD8 antibody (MBL), PE-labeled H-2Kb OVA Tetramer-SIINFEKL (MBL), and Fixable Viability dye eFluor 520 (invitrogen). The data were taken and analyzed with FACS Cant II (BD Biosciences) and FLOWJO software (TreeStar). The results are shown in Figures 5 and 6.

Further separately, the spleen cell was stained with V450-labeled antimouse CD3e antibody (invitrogen), Alexa Fluor^{™} 647-labeled antimouse CD8 antibody (MBL), PE-Cy7-labeled antimouse CD44 antibody (Invitrogen), PerCP-Cy5.5-labeled antimouse CD62L antibody (Invitrogen), and Fixable Viability dye eFluor 520 (Invitrogen). The data were taken and analyzed with FACS Cant II (BD Biosciences) and FLOWJO software (TreeStar). The results are shown in Figures 7 and 8.

By using the compound of Example 1, Example 2, or Reference example 9, the percentage of OVA-specific type 1 helper T-cell, especially OVA-specific multifunctional CD4-positive T-lymphocyte, the percentage of MHC-restricted OVA-specific CD8-positive T-lymphocyte (OVA tetramer-positive CD8T cell in Figures 5 and 6), and the percentage of CD8-positive effector memory T-lymphocyte were significantly increased, compared with the negative control group.

The results in Tests 3 and 4 have clarified that the example compounds of the present invention have adjuvant effect.

### INDUSTRIAL APPLICABILITY

The compounds of the present invention are useful as adjuvant for enhancing immunostimulating activity in a vaccine formulation.

## Claims

1. A compound of formula (1): 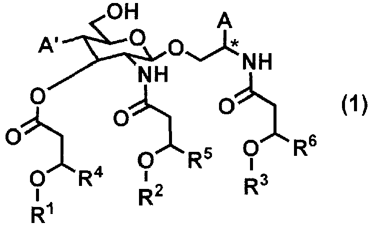 or a pharmaceutically acceptable salt thereof, wherein
A and A' are independently hydrogen, hydroxy, or - (CH₂)ₘ-COOH, provided that at least one of A or A' is - (CH₂)ₘ-COOH,
R¹ is -C(O)(CH₂)ₙ-X or -CH₂-(CH₂)ₙ-X,
R² is -C(O)(CH₂)ₒ-Y or -CH₂-(CH₂)ₒ-Y,
R³ is -C(O)(CH₂)ₚ-Z or -CH₂-(CH₂)ₚ-Z,
X, Y, and Z are independently methyl, C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy), or 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy), provided that at least one of X, Y, or Z is C₆₋₁₀ aryl (said C₆₋₁₀ aryl may be substituted with 1 - 5 substituents selected independently from hydroxy, C₁₋₆ alkyl, halogen, cyano, and C₁₋₆ alkoxy) or 5- to 10-membered heteroaryl (said 5- to 10-membered heteroaryl may be substituted with 1 - 4 substituents selected independently from hydroxy, C₁₋₆ alkyl, halogen, cyano, and C₁₋₆ alkoxy),
provided that when A is -COOH and the stereochemistry of * is S-configuration, Y is methyl,
R⁴, R⁵, and R⁶ are independently C₁₀₋₂₀ alkyl,
m is independently an integer of 0 - 6, and
n, o, and p are independently an integer of 5 - 20.

2. The compound of claim 1, or a pharmaceutically acceptable salt thereof, wherein m is 0.

3. The compound of claim 1 or 2, or a pharmaceutically acceptable salt thereof, wherein R¹ is -C(O)(CH₂)ₙ-X, R² is -C(O) (CH₂)ₒ-Y, and R³ is -C(O) (CH₂)ₚ-Z.

4. The compound of any one of claims 1 to 3, or a pharmaceutically acceptable salt thereof, wherein A is COOH.

5. The compound of any one of claims 1 to 4, or a pharmaceutically acceptable salt thereof, wherein A is COOH, and A' is hydroxy.

6. The compound of any one of claims 1 to 5, or a pharmaceutically acceptable salt thereof, wherein R⁴, R⁵, and R⁶ are independently C₁₀₋₁₂ alkyl.

7. The compound of claim 1 which is represented by formula (2): 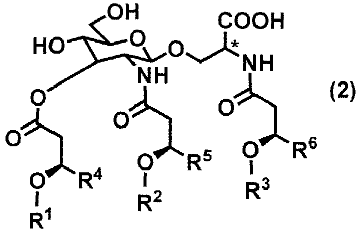 wherein
R¹ is -C(O)(CH₂)ₙ-X,
R² is -C(O)(CH₂)ₒ-Y,
R³ is -C(O)(CH₂)ₚ-Z,
X, Y, and Z are independently methyl, C₆₋₁₀ aryl, or 5-to 10-membered heteroaryl, provided that at least one of X, Y, or Z is C₆₋₁₀ aryl or 5- to 10-membered heteroaryl,
provided that when the stereochemistry of * is S-configuration, Y is methyl,
R⁴, R⁵, and R⁶ are independently C₁₀₋₁₂ alkyl, and
n, o, and p are independently an integer of 6 - 10,
or a pharmaceutically acceptable salt thereof.

8. The compound of claim 1 which is represented by formula (3): 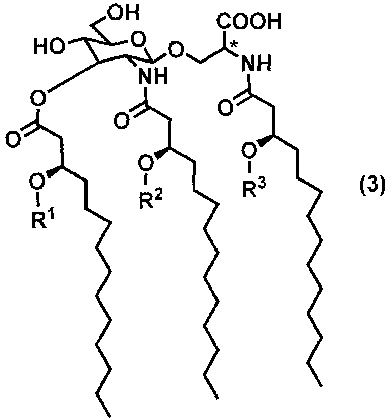 wherein
R¹ is -C(O)(CH₂)ₙ-X,
R² is -C(O)(CH₂)ₒ-Y,
R³ is -C(O)(CH₂)ₚ-Z,
X, Y, and Z are independently methyl, C₆₋₁₀ aryl, or 5-to 10-membered heteroaryl, provided that at least one of X, Y, or Z is C₆₋₁₀ aryl or 5- to 10-membered heteroaryl,
provided that when the stereochemistry of * is S-configuration, Y is methyl, and
n, o, and p are independently an integer of 6 - 10,
or a pharmaceutically acceptable salt thereof.

9. The compound of claim 1 which is represented by formula (4) or formula (5): 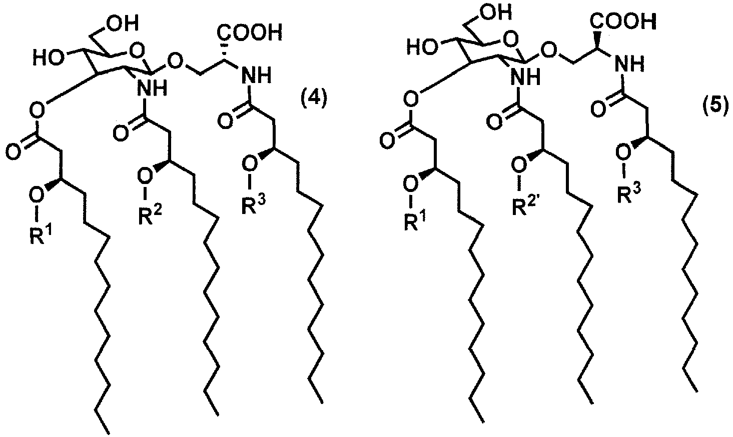 wherein
R¹ is -C(O)(CH₂)ₙ-X,
R² is -C(O)(CH₂)ₒ-Y,
R³ is -C(O)(CH₂)ₚ-Z,
R^{2'} is -C(O)(CH₂)ₒ-CH₃,
X, Y, and Z are independently methyl, C₆₋₁₀ aryl, or 5-to 10-membered heteroaryl, provided that at least one of X, Y, or Z in formula (4) is C₆₋₁₀ aryl or 5- to 10-membered heteroaryl, and at least one of X or Z in formula (5) is C₆₋₁₀ aryl or 5- to 10-membered heteroaryl, and
n, o, and p are independently an integer of 7 - 9,
or a pharmaceutically acceptable salt thereof.

10. The compound of any one of claims 1 to 9, or a pharmaceutically acceptable salt thereof, wherein
X, Y, and Z are independently methyl or phenyl, provided that at least one of X, Y, or Z is phenyl,
provided that when A is -COOH and the stereochemistry of * is S-configuration, Y is methyl.

11. The compound of claim 1 which is selected from the following compound group:
(2*R*)-2-{[(3*R*)-3-(decanoyloxy)tetradecanoyl]amino}-3-{[3-{[(3*R*)-3-(decanoyloxy)tetradecanoyl]amino}-5-hydroxy-6-(hydroxymethyl)-4-({(3*R*)-3-[(9-phenylnonanoyl)oxy]tetradecanoyl}oxy)oxan-2-yl]oxy}propanoic acid,
(2*S*)-2-{[(3*R*)-3-(decanoyloxy)tetradecanoyl]amino}-3-{[3-{[(3*R*)-3-(decanoyloxy)tetradecanoyl]amino}-5-hydroxy-6-(hydroxymethyl)-4-({(3*R*)-3-[(9-phenylnonanoyl)oxy]tetradecanoyl}oxy)oxan-2-yl]oxy}propanoic acid,
(2*R*)-2-{[(3*R*)-3-(decanoyloxy)tetradecanoyl]amino}-3-{[4-{[(3*R*)-3-(decanoyloxy)tetradecanoyl]oxy}-5-hydroxy-6-(hydroxymethyl)-3-({(3*R*)-3-[(9-phenylnonanoyl)oxy]tetradecanoyl}amino)oxan-2-yl]oxy}propanoic acid,
(2*R*)-2-({[(3*R*)-3-(9-phenylnonanoyl)oxy]tetradecanoyl}amino)-3-{[3-{[(3*R*)-3-(decanoyloxy)tetradecanoyl]amino}-5-hydroxy-6-(hydroxymethyl)-4-({(3*R*)-3-(decanoyloxy)tetradecanoyl}oxy)oxan-2-yl]oxy}propanoic acid, and
(2*S*)-2-(3*R*)-3-(9-phenylnonanoyl)oxy]tetradecanoyl}amino)-3-{[3-{[(3*R*)-3-(decanoyloxy)tetradecanoyl]amino}-5-hydroxy-6-(hydroxymethyl)-4-({ (3*R*)-3-(decanoyloxy)tetradecanoyl}oxy)oxan-2-yl]oxy}propanoic acid.

12. A pharmaceutical composition comprising the compound of any one of claims 1 to 11, or a pharmaceutically acceptable salt thereof.

13. The pharmaceutical composition of claim 12, which is a lipid formulation.

14. The pharmaceutical composition of claim 12 or 13, wherein the lipid formulation is a liposome formulation including phospholipid.

15. The pharmaceutical composition of claim 14, wherein the phospholipid is 1,2-dimyristoyl-sn-glycero-3-phosphocholine and egg yolk phosphatidylglycerol.

16. The pharmaceutical composition of claim 14 or 15, wherein the lipid formulation comprises at least one additive selected form the group consisting of an inorganic acid, an inorganic acid salt, an organic acid, an organic acid salt, sugars, a buffering agent, an antioxidant, and polymers.

17. The pharmaceutical composition of any one of claims 12 to 16, which further comprises an antigen.

18. The pharmaceutical composition of claim 17, wherein the antigen is a pathogen-derived antigen.

19. A vaccine adjuvant comprising the compound of any one of claims 1 to 11, or a pharmaceutically acceptable salt thereof.

20. The vaccine adjuvant of claim 19, which is an adjuvant for infection vaccine.

21. A kit comprising
a) the compound of claim 1 or a pharmaceutically acceptable salt thereof, or a pharmaceutical composition comprising the compound of claim 1 or a pharmaceutically acceptable salt thereof; and
b) a pharmaceutical composition comprising an antigen.

22. The kit of claim 21, wherein the antigen is a pathogen-derived antigen.
